# FASCICULE DE BREVET EUROPEEN

(11) **EP 1 352 633 B1**
(45) Date de publication et mention de la délivrance du brevet: **08.03.2006**
(21) Numéro de dépôt: 03290750.3
(22) Date de dépôt: 25.03.2003
(51) Int. Cl.: A61K 8/22, A61K 8/36, A61K 8/362, A61K 8/365, A61K 8/368, A61K 8/38, A61K 8/41, A61Q 5/10, A61Q 7/00

(54) **Kit pour la teinture des fibres kératiniques**
Kit zur Färbung von keratinischen Fasern
Kit for dying keratinic fibers

(30) Priorité: 11.04.2002 FR 0204529
(43) Date de publication de la demande: 15.10.2003
(73) Titulaire: L'OREAL, 75008 Paris (FR)
(72) Inventeur: Quinn, Francis Xavier, 75005 Paris (FR); Giustiniani, Pascal, 92300 Levallois Perret (FR)
(74) Mandataire: Fevrier, Murielle Françoise E.

(56) Documents cités:
- EP-A- 0 850 638
- WO-A-99/37276
- FR-A- 2 615 730

## Description

L'invention concerne un kit de teinture des fibres kératiniques, en particulier des cheveux humains, comprenant une composition alcalinisante et une composition oxydante. L'invention concerne aussi la composition oxydante du kit, la composition prête à l'emploi obtenue à partir de ce kit ainsi que le procédé de teinture mettant en oeuvre ce kit.

II est connu de teindre les fibres kératiniques et en particulier les cheveux humains par coloration d'oxydation avec des compositions tinctoriales contenant des précurseurs de colorant d'oxydation, généralement appelés bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Le procédé de teinture par coloration d'oxydation, coloration dite "permanente", consiste à appliquer sur le fibres kératiniques une composition comprenant des bases d'oxydation ou un mélange de bases d'oxydation et de coupleurs, et un agent oxydant, par exemple du peroxyde d'hydrogène, à laisser pauser, puis à rincer les fibres.

Les colorations qui en résultent sont permanentes, puissantes, et résistantes aux agents extérieurs, notamment à la lumière, aux intempéries, aux lavages, à la transpiration et aux frottements. Généralement appliquées à pH basique, le procédé permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris et, dans le cas de cheveux naturellement pigmentés, de faire ressortir la couleur, c'est à dire de la rendre plus visible.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres. Les colorants directs classiques sont par exemple des colorants du type nitrés benzèniques, anthraquinoniques, nitropyridiniques, azoïques, méthiniques, azométhiniques, xanthéniques, acridiniques aziniques ou des colorants triarylméthane.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Il est également connu d'utiliser des colorants directs en combinaison avec des agents oxydants et des agents alcalins pour obtenir des teintures directes éclaircissantes.

Par exemple, la demande de brevet FR 2 741 798 décrit des compositions tinctoriales contenant des colorants directs comportant au moins un atome d'azote quatemisé du type azoïque ou azométhine, lesdites compositions étant à mélanger extemporanément à pH basique à une composition oxydante. Ces compositions permettent d'obtenir des colorations avec des reflets homogènes, tenaces et brillants.

Il est aussi connu dans les demandes de brevet EP 850 638, EP 852 135, EP 850 637, EP 850 636, d'utiliser des colorants directs en teinture d'oxydation en associant un colorant direct cationique avec une base d'oxydation.

Cependant, les colorants directs sont généralement peu stables dans ces types de compositions, en particuliers en solution alcaline ou oxydante ce qui les rendent difficilement utilisables dans les compositions de teinture directe éclaircissante à base d'eau oxygénée et d'un agent alcalinisant ou dans des compositions de teinture d'oxydation en association avec des précurseurs du type bases d'oxydation ou coupleurs.

Dans de tels milieux, les colorants directs cationiques ont tendances à se dégrader ce qui limite la durée de conservation des compositions tinctoriales ainsi que la capacité de coloration de ces compositions.

Le but de la présente invention est de foumir un système permettant de stabiliser les colorants directs cationiques desbnés à être utilisés en coloration d'oxydation ou dans des teintures éclaircissantes tout en conservant leur affinité pour les fibres kératiniques ainsi que leur pouvoir colorant.

Ce but est atteint avec la présente invention qui a pour objet un kit pour la teinture de fibres kératiniques comprenant
- une composition alcalinisante comprenant, dans un milieu approprié pour la teinture, un agent alcalin, et
- une composition oxydante ayant un pH inférieur ou égal à 7 comprenant, dans un milieu approprié, un agent oxydant et un complexe moléculaire en suspension dans le milieu, le complexe étant formé d'un colorant direct et d'un acide organique insoluble
ou faiblement soluble dans la composition oxydante dont le pKa est supérieur au pH de la composition oxydante et inférieur au pH du mélange de la composition oxydante et de la composition alcalinisante, à la condition que l'écart entre le pKa de l'acide et le pH de la composition oxydante est d'au moins une unité et l'écart entre le pKa de l'acide et le pH du mélange est d'au moins une unité.

La présente invention a aussi pour objet une composition oxydante telle que définie précédemment, ainsi qu'une composition prête à l'emploi qui comprend en mélange une composition oxydante et une composition atcalinisante telles que définies précédemment.

La présence du colorant direct cationique sous forme d'un complexe moléculaire permet d'éviter la dégradation du colorant, l'acide jouant un rôle protecteur vis à vis de l'agent oxydant. On obtient ainsi une composition stable au stockage dont les propriétés tinctoriales ne sont pas dégradées dans le temps.

Au moment de l'utilisation, les compositions oxydantes et alcalinisantes sont mélangées pour former une composition prête à l'emploi. Il s'ensuit une modification du pH ce qui entraîne la dissolution du complexe et la libération du colorant direct cationique dans le milieu, colorant qui est alors disponible pour la teinture.

Le kit de l'invention est particulièrement utile en coloration d'oxydation ou en coloration directe éclaircissante.

Dans le kit de la présente invention, le pH de la composition alcalinisante est de préférence compris entre 7 et 12 et le pH de la composition oxydante est de préférence inférieur ou égal à 5.

Dans le cadre de l'invention, un acide organique insoluble ou faiblement soluble dans la composition oxydante est un acide dont la solubilité dans cette composition à 15°C est inférieure à 10 g/l, de préférence inférieure à 5 g/l, de façon plus préférée 1g/l.

L'acide organique insoluble formant le complexe moléculaire est par exemple choisi parmi l'acide chenodeoxycholique, l'acide benzoïque, l'acide cinnamique, l'acide cholique et l'acide desoxycholique.

Le colorant direct peut être choisi parmi les colorants directs neutres, cationiques ou anioniques. De préférence, le colorant direct utile dans la composition de la présente invention est un colorant direct cationique.

Les colorants directs utiles dans la présente invention sont des colorants directs cationiques, de préférence porteur d'un groupe ammonium quatemaire, qui sont par exemple choisis parmi les amino-anthraquinoniques cationiques, les mono- ou di-azoïques cationiques, les méthiniques cationiques, les azométhiniques cationiques ou les naphtoquinones cationiques. Les colorants directs préférés sont choisi parmi les colorants azoïques (-N=N-), azométhiniques (-N=C-) ou méthiniques (-C=C-) comportant un groupe ammonium quaternaire

Selon un mode de réalisation particulier, le colorant direct cationique est un composé de formule (I) suivante : 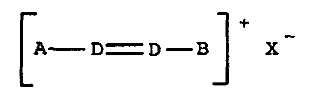
dans laquelle, D désigne un atome d'azote ou un radical CH , A et B désignent des groupements aromatiques pouvant être formés d'un cycle aromatique ayant de 4 à 6 chaînons ou de plusieurs cycles aromatiques condensés, le ou les cycles aromatiques pouvant contenir un ou plusieurs hétéroatomes et pouvant être substitués par un ou plusieurs atomes d'halogène ou par un ou plusieurs radicaux choisis parmi NR₁₁R₁₂, ou OR₁₁, dans lesquels R₁₁ et R₁₂, simultanément ou indépendamment l'un de l'autre, représentent l'hydrogène, un radical alkyle en C₁-C₈, un radical hydroxyalkyfe en C₁₋C₄, ou un radical phényle, et X désigne un anion, à la condition qu'au moins un des groupes A ou B comporte un groupement cationique, de préférence un groupement ammonium quaternaire.

Le groupement cationique peut faire partie intégrante du groupement aromatique ou être porté par l'un de ses substituants.

Dans le cadre de l'invention, le radical alkyle peut être linaire ou ramifié, substitué ou non substitué. Le groupement aromatique est par exemple un groupe phényle, naphtyle, pyridine, pyrimidine, imidazole, pyrazole, pipérazine, pyrrolidine, pyrrole, pipéridine, imidazolidine, etc.

A titre de d'exemple de colorant direct cationique, on peut notamment citer le chlorure de [8-[(p-aminophényl)azol]-7-hydroxy-2-naphtyl]triméthylammonium (également appelé Basic Brown 16 ou Arianor Mahogany 306002 dans le Color Index), le chlorure de 3-[(4-amino-6-bromo-5,8-dihydro-1-hydroxy-8-imino-5-oxo-2-naphtalényl)-amino]-N,N,N-triméthyl-benzénaminium (également dénommé Basic Blue 99 ou Arianor Steel Blue 306004 dans le Color Index), le chlorure de 7-hydroxy-8-[(2-méthoxyphényl)azo]-N,N,N-triméthyl-2-naphtalènaminium (également appelé le Basic Red 76 ou Arianor Madder Red dans le Color Index), le chlorure de [8-[(4-amino-2-nitrophényl)azo]-7-hydroxy-2-naphtyl]triméthylammonium (également appelé Basic Brown 17 ou Arianor Sienna Brown 306001 dans le Color Index) et le chlorure de 3-[(4,5-dihydro-3-méthyl-5-oxo-1-phényl-1H-pyrazol-4-yl)azo]-N,N,N-triméthylbenzènaminium également appelé Basic Yellow 57 ou Arianor Straw Yellow 306005 dans le Color Index).

Selon un premier mode de réalisation, le colorant direct est choisi parmi les composés de formule (V) suivante : 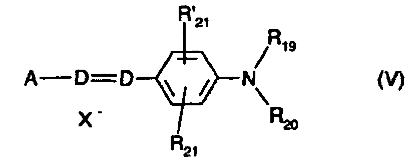
dans laquelle :
- D représente un atome d'azote ou le groupement -CH,
- R₁₉ et R₂₀, identiques ou différents, représentent un atome d'hydrogène ; un radical alkyle en C₁-C₄ pouvant être substitué par un radical -CN, -OH ou -NH₂ ; un radical 4'-aminophényle ; ou forment avec un atome de carbone du cycle benzénique un hétérocycle éventuellement oxygéné et pouvant être substitué par un ou plusieurs radicaux alkyle en C₁-C₄ ;
- R₂₁ et R'₂₁, identiques ou différents, représentent un atome d'hydrogène ou d'halogène choisi parmi le chlore, le brome, l'iode et le fluor, un radical cyano, alkyle, alcoxy en C₁-C₄ ou acétyloxy,
- X est un anion,
- A représente un groupement choisi par les structures A1 à A19 suivantes : 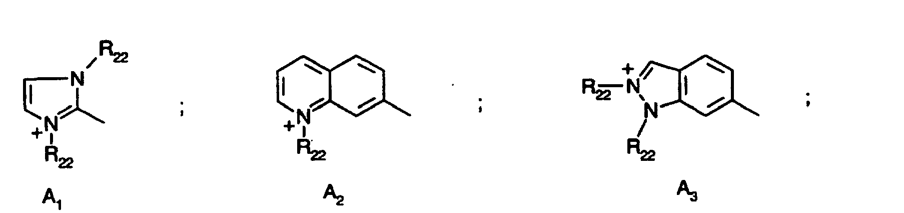 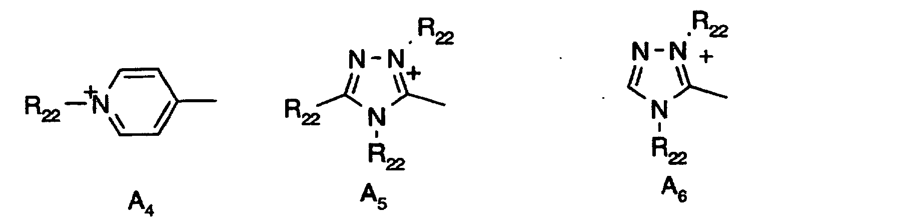 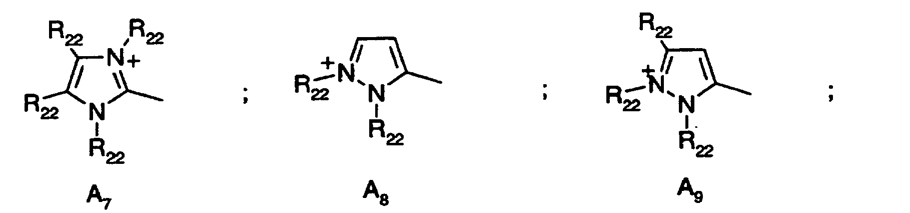 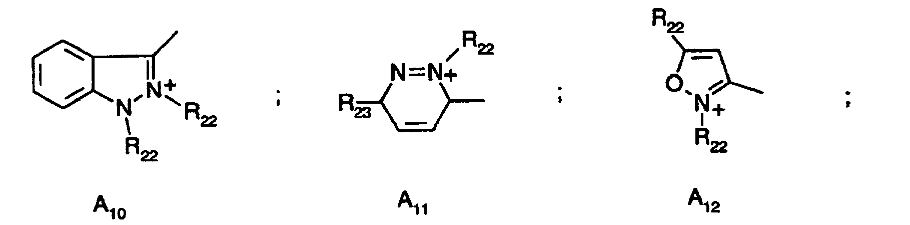 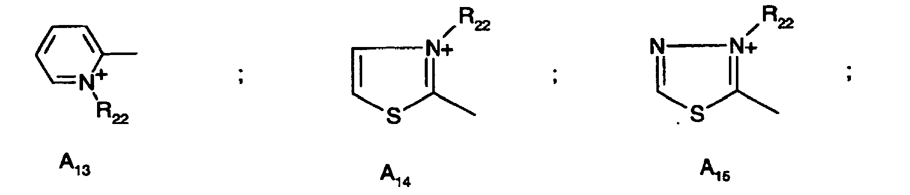 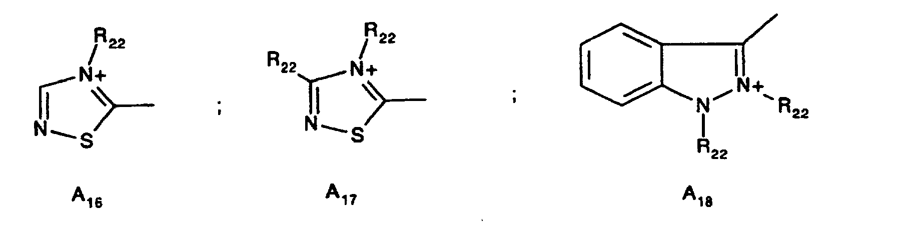 et 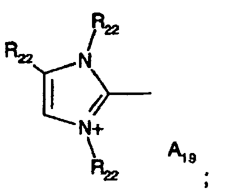
   dans lesquelles
- R₂₂ représente un radical alkyle en C₁-C₄ pouvant être substitué par un radical hydroxyle et R₂₃ représente un radical alcoxy en C₁-C₄.

Selon un mode de réalisation particulier, A est A1, R₁₉ et R₂₀, identiques ou différents, représentent un atome d'hydrogène, un radical alkyle en C₁-C₄ ou un radical 4'-aminophényle et R₂₂ représente un radical alkyle en C₁-C₄

Selon un deuxième mode de réalisation, le colorant direct cationique est choisi parmi les composés de formule (VI) suivante : 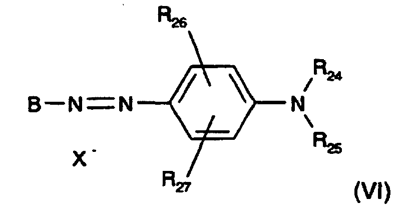
dans laquelle :
- R₂₄ représente un atome d'hydrogène ou un radical alkyle en C₁-C₄,
- R₂₅ représente un atome d'hydrogène, un radical alkyle pouvant être substitué par un radical -CN ou par un groupement amino, un radical 4'-aminophényle ou forme avec R₂₄ un hétérocycle éventuellement oxygéné pouvant être substitué par un radical alkyle en C₁-C₄,
- R₂₆ et R₂₇, identiques ou différents, représentent un atome d'hydrogène, un atome d'halogène tel que le brome, le chlore, l'iode ou le fluor, un radical alkyle en C₁-C₄ ou alcoxy en C₁-C₄, un radical-CN,
- X est un anion,
- B représente un groupement choisi par les structures B1 à B6 suivantes :

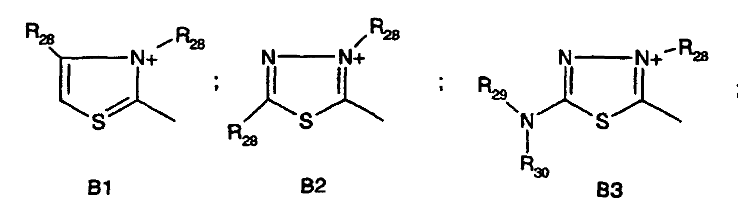

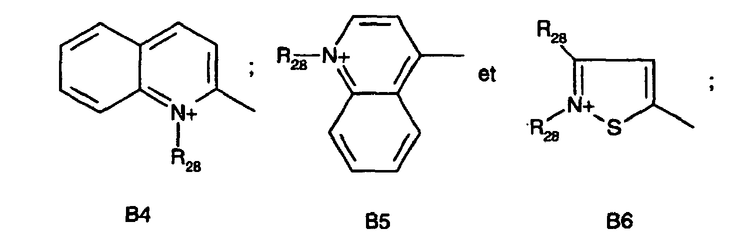

dans lesquelles R₂₈ représente un radical alkyle en C₁-C₄, R₂₉ et R₃₀, identiques ou différents, représentent un atome d'hydrogène ou un radical alkyle en C₁-C₄.

Selon un troisième mode de réalisation, le colorant direct cationique est choisi parmi les composés de formules (VII) et (VII') suivantes : 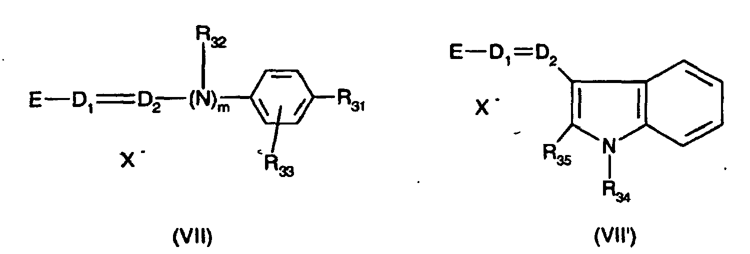
dans lesquelles :
- R₃₁ représente un atome d'hydrogène, un radical alcoxy en C₁-C₄, un atome d'halogène tel que le brome, le chlore, l'iode ou le fluor ou un radical amino,
- R₃₂ représente un atome d'hydrogène, un radical alkyle en C₁-C₄ ou forme avec un atome de carbone du cycle benzénique un hétérocycle éventuellement oxygéné pouvant être substitué par un ou plusieurs groupements alkyle en C₁-C₄,
- R₃₃ représente un atome d'hydrogène ou d'halogène tel que le brome, le chlore, l'iode ou le fluor,
- R₃₄ et R₃₅, identiques ou différents, représentent un atome d'hydrogène ou un radical alkyle en C₁-C₄,
- D₁ et D₂, identiques ou différents, représentent un atome d'azote ou le groupement - CH,
- m = 0 ou 1,
   étant entendu que lorsque R₃₁ représente un groupement amino non substitué, alors D₁ et D₂ représentent simultanément un groupement -CH et m = 0,
- X est un anion,
- E représente un groupement choisi par les structures E1 à E8 suivantes :

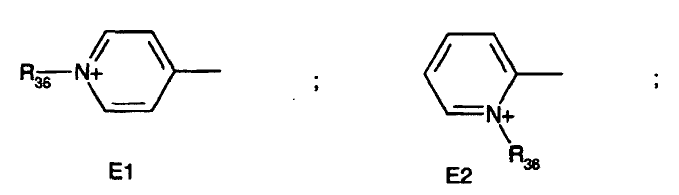

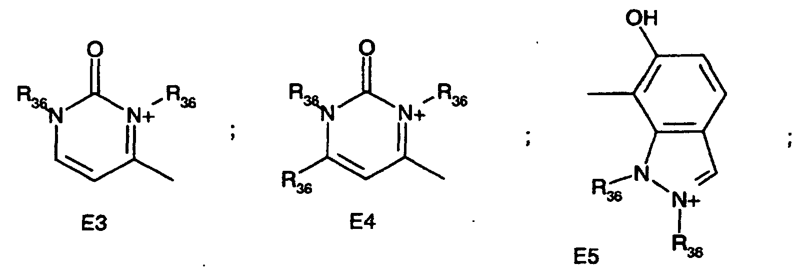

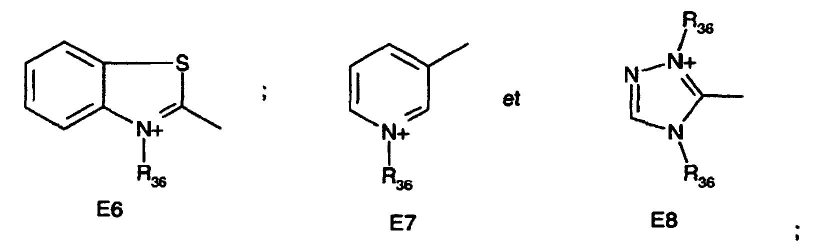

dans lesquelles R₃₆ représente un radical alkyle en C₁-C₄ ;
lorsque m = 0 et que D₁ représente un atome d'azote, alors E peut également désigner un groupement de structure E9 suivante :

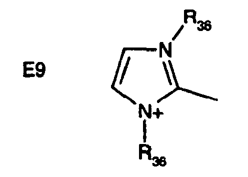

Le composé de formule (VII) dans lequel m = 1, R₃₂ représente un atome d'hydrogène ou un radical alkyle et E représente E1 est particulièrement préféré.

Les colorants directs cationiques de formules (V), (VI), (VII) et (VII') utiles dans la présente invention sont des composés connus et sont décrits par exemple dans les demandes de brevets WO 95/01772, WO 95/15144 et EP-A-0 714 954.

Parmi les colorants directs cationiques de formule (V), on peut plus particulièrement citer les composés répondant aux structures (V1) à (V52) suivantes :

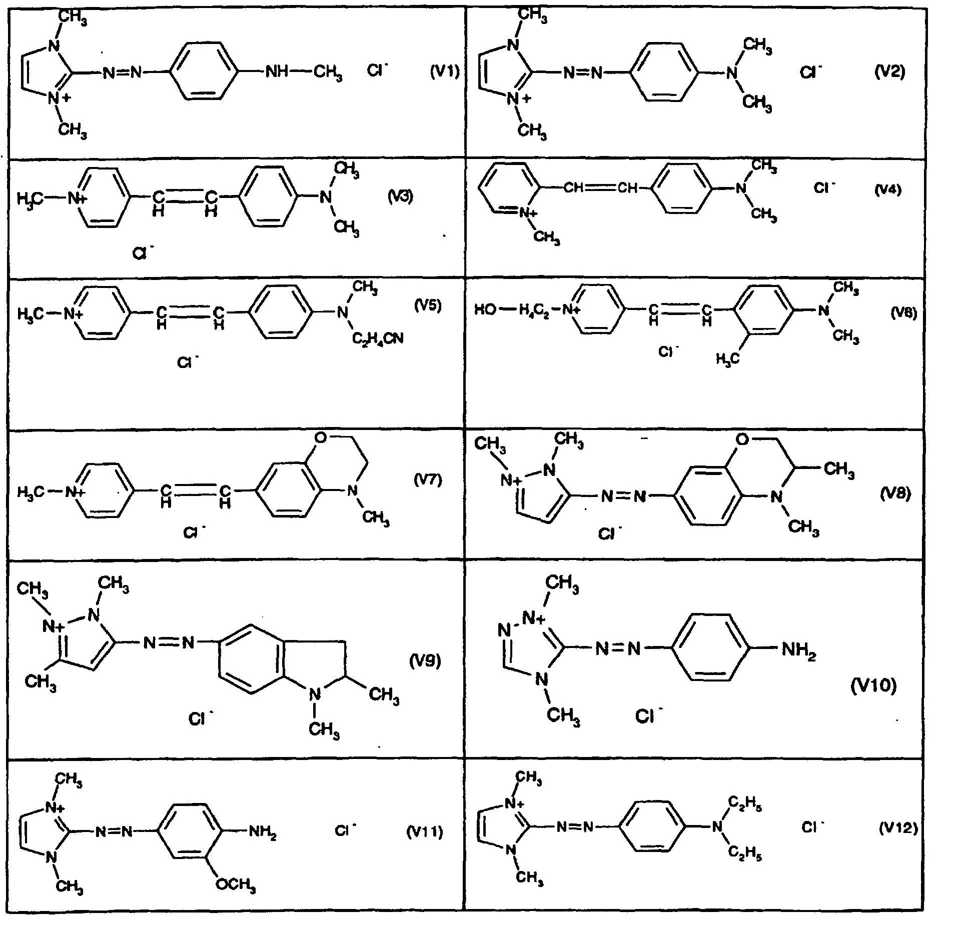

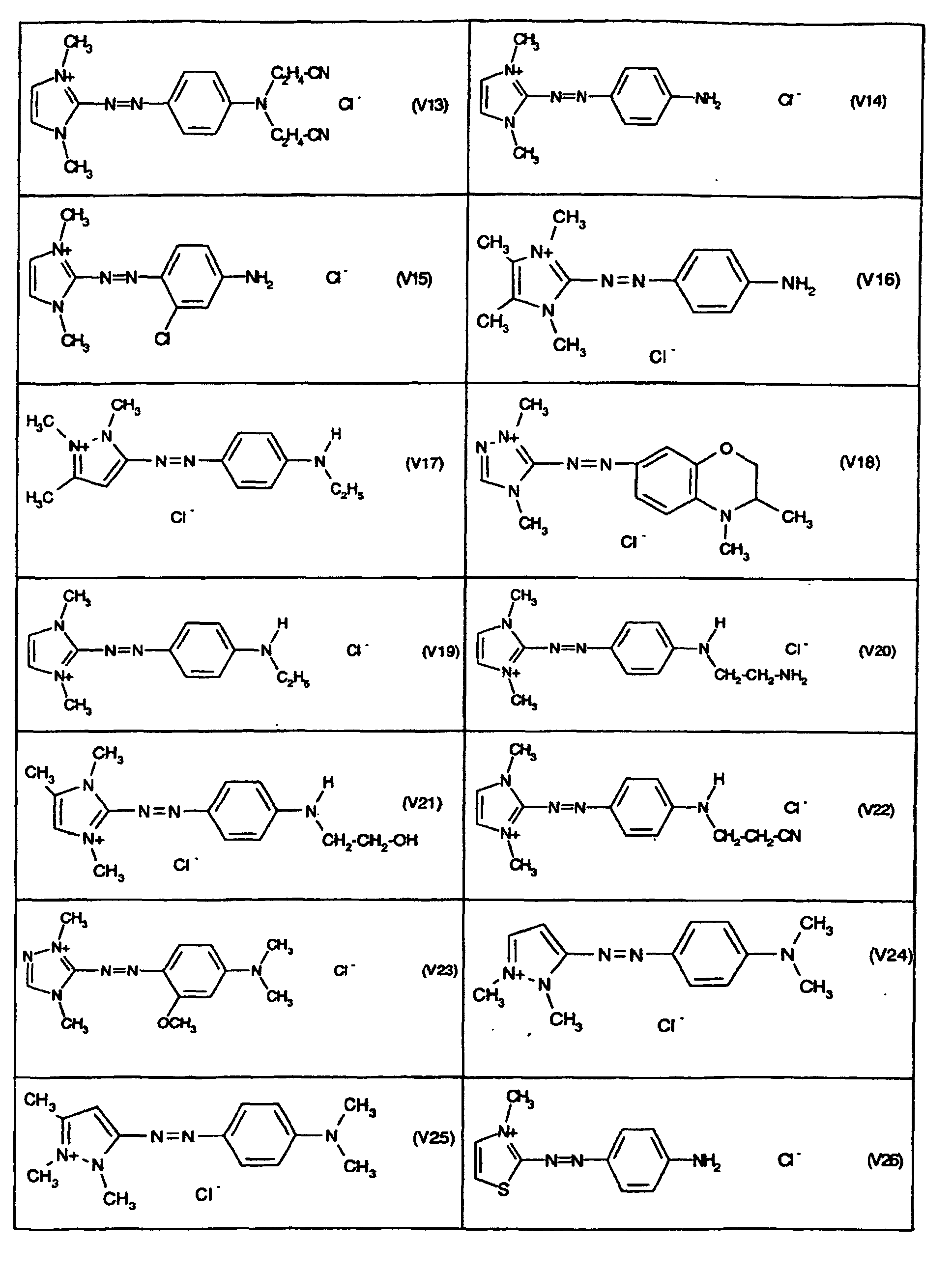

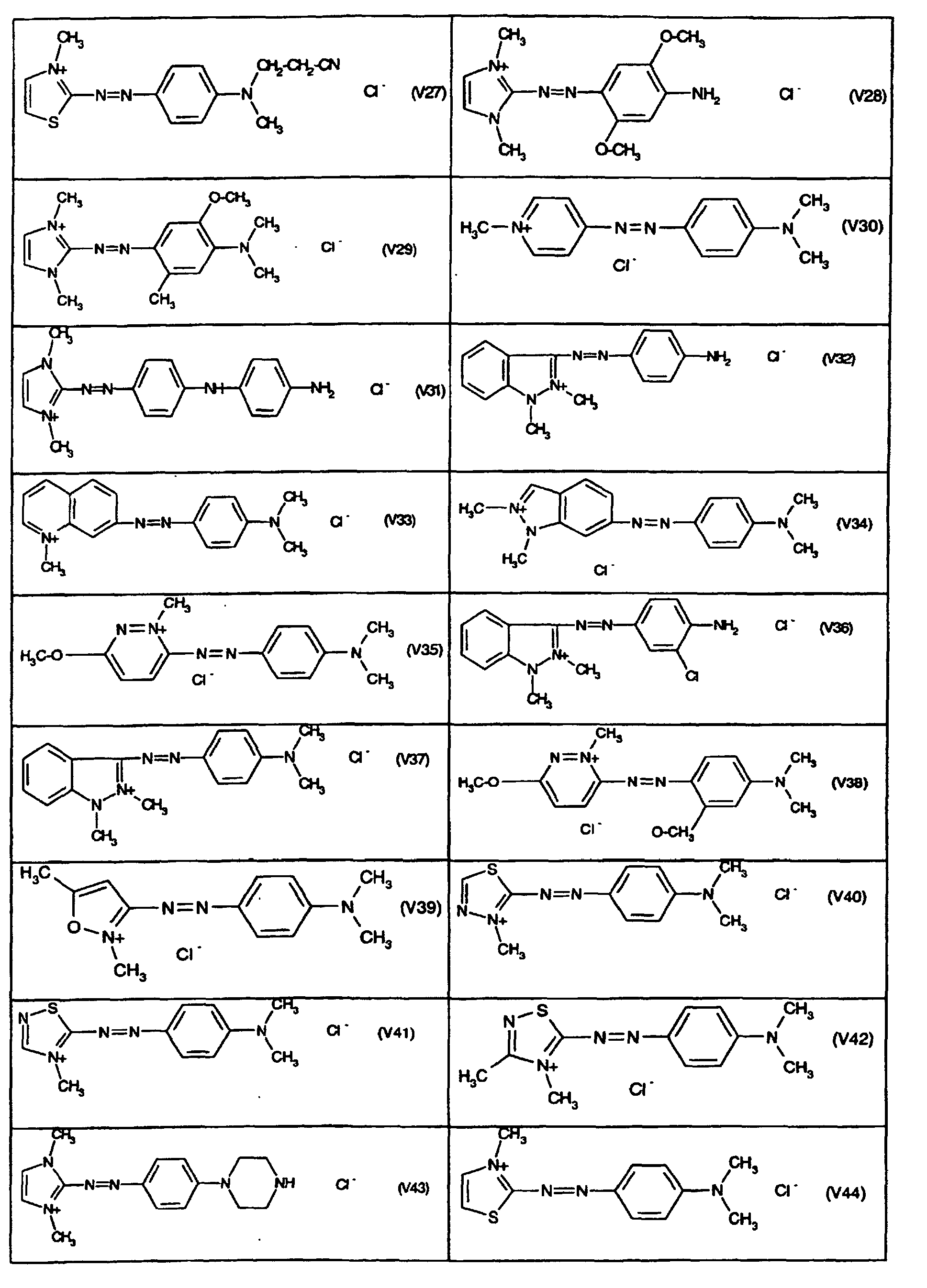

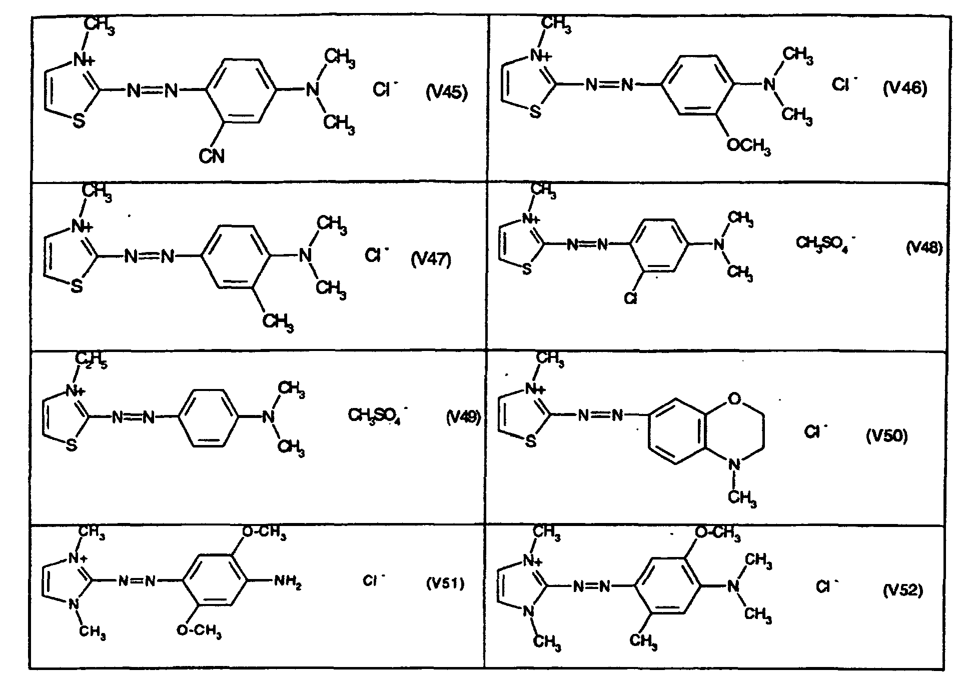

Parmi les composés de structures (V1) à (V52) décrits ci-dessus, on préfère tout particulièrement les composés répondant aux structures (V1), (V14) et (V31).

Parmi les colorants directs cationiques de formule (VI), on peut plus particulièrement citer les composés répondant aux structures (VI1) à (VI12) suivantes :

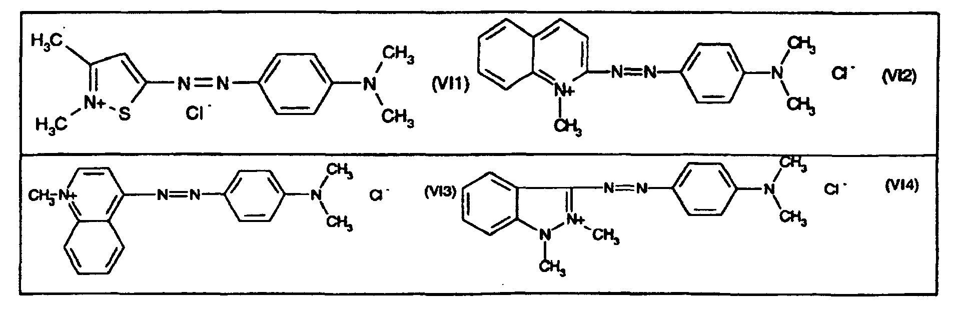

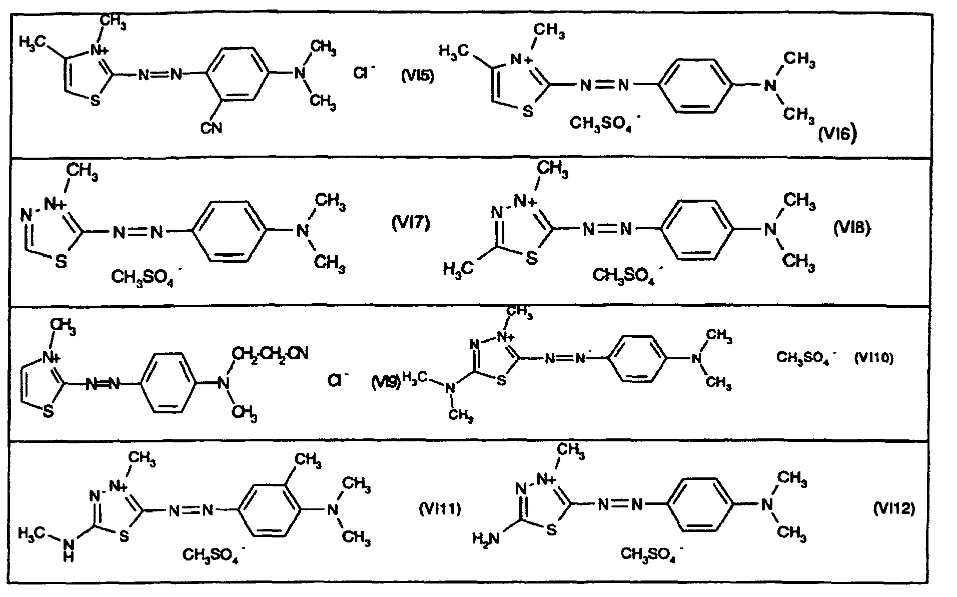

Parmi les colorants directs cationiques de formule (VII), on peut plus particulièrement citer les composés répondant aux structures (VII1) à (VII18) suivantes :

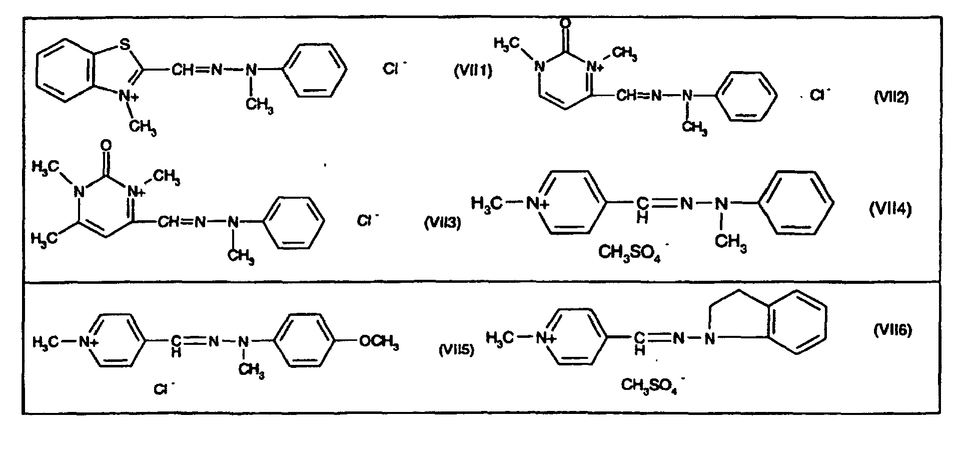

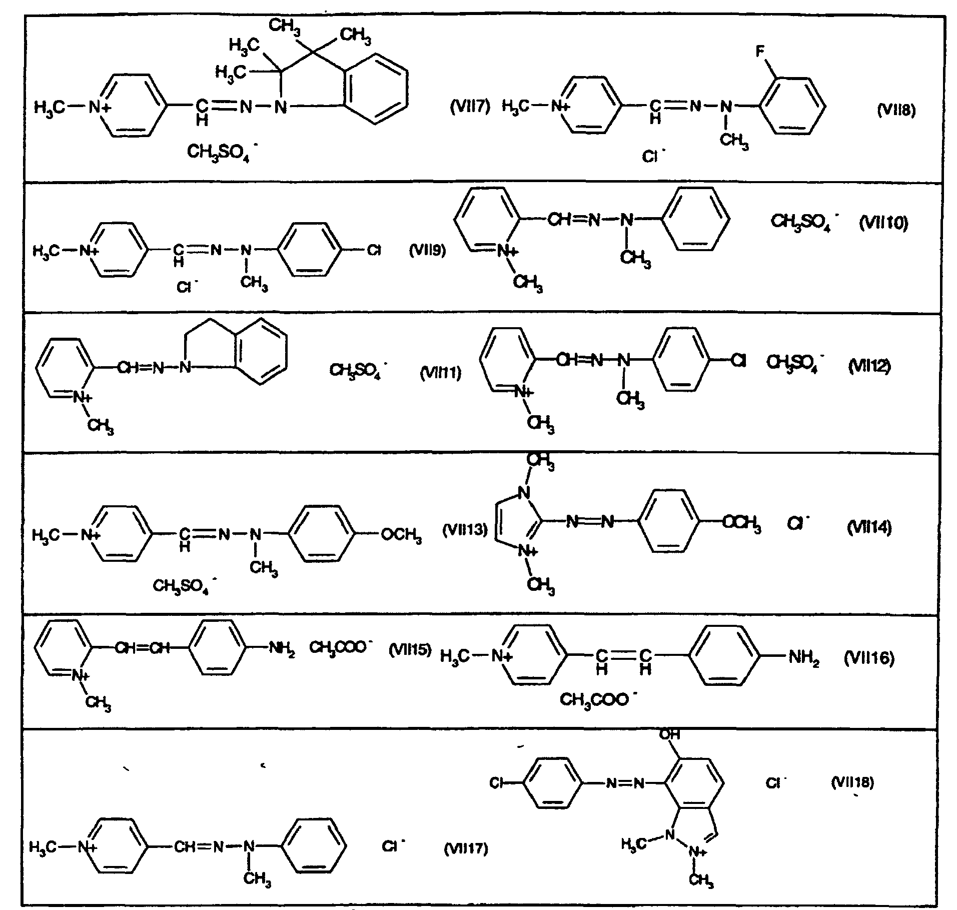

Parmi les composés particuliers de structures (VII1) à (VII18) décrits ci-dessus, on préfère tout particulièrement les composés répondant aux structures (VII4), (VII5) et (VII13).

Parmi les colorants directs cationiques de formule (VII'), on peut plus particulièrement citer les composés répondant aux structures (VII'1) à (VII'3) suivantes :

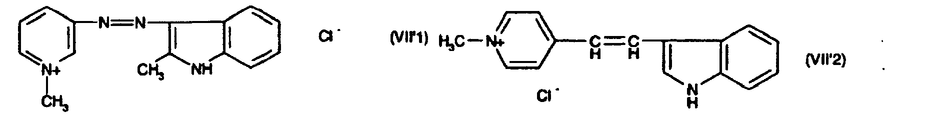

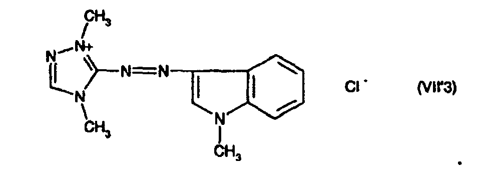

D'autres colorants directs cationiques utiles dans le cadre de la présente invention sont décrits dans les demandes de brevets FR 2 189 006, FR 2 285 851 et FR 2 140 205. A titre d'exemple, on peut citer plus particulièrement les composés de structures (I)₁ à (I)₇₇ suivantes :

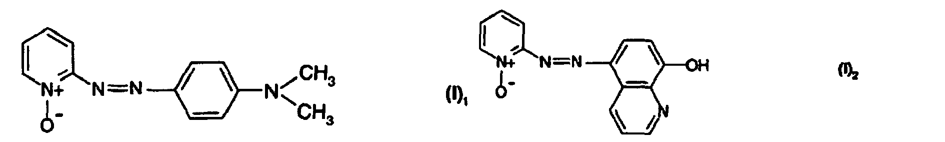

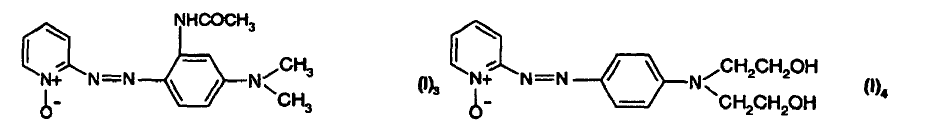

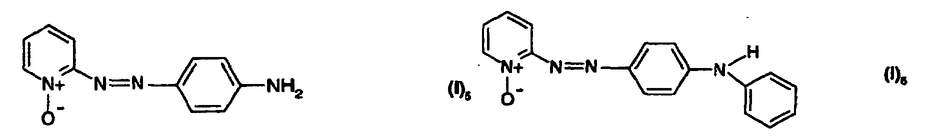

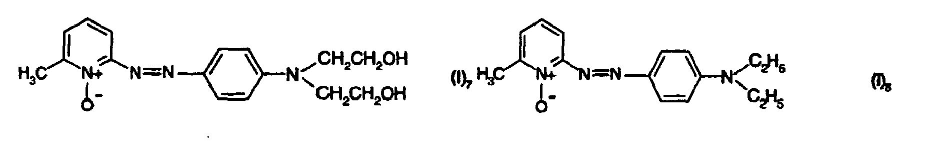

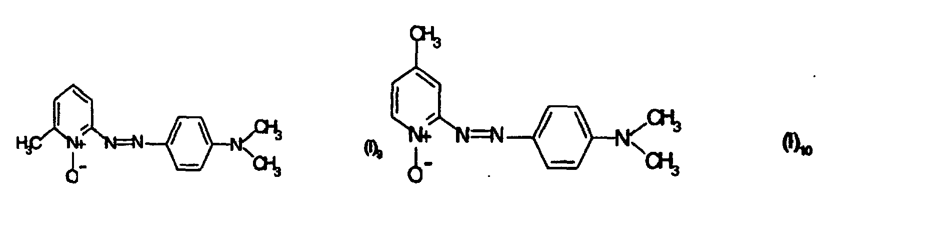

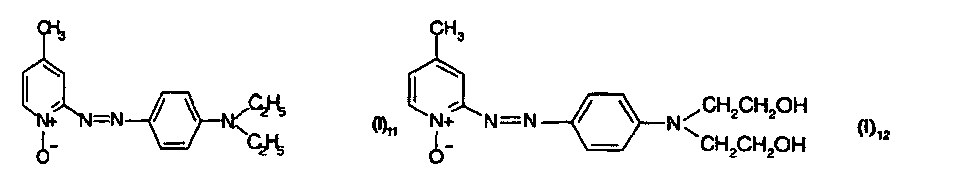

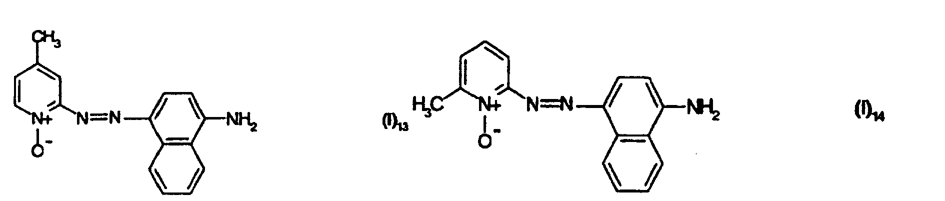

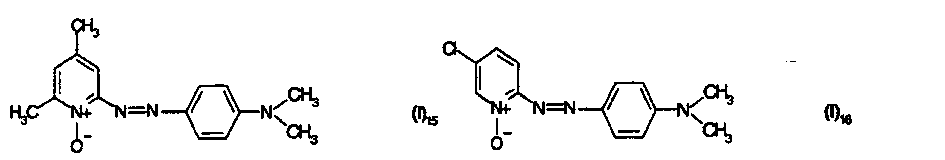

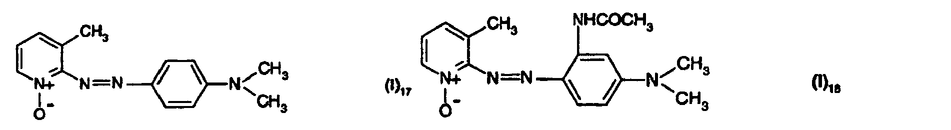

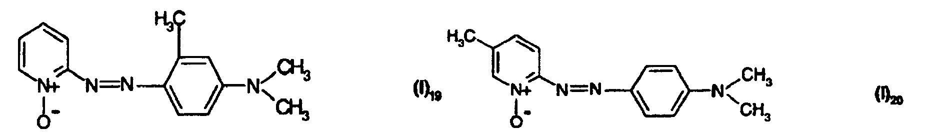

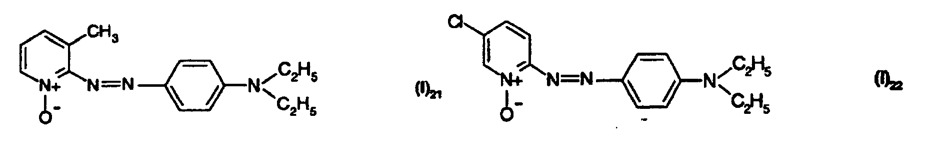

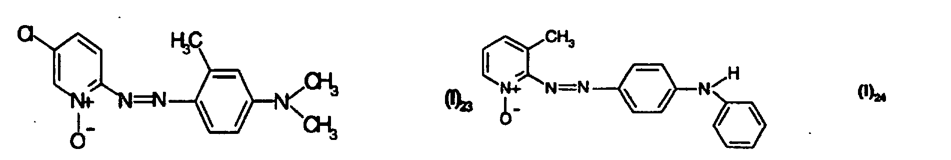

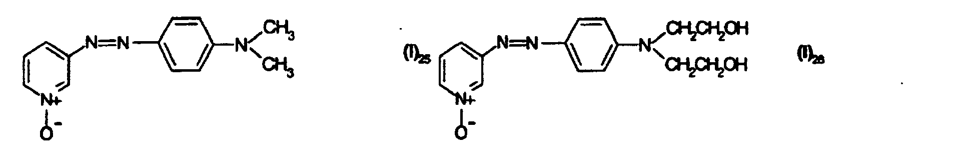

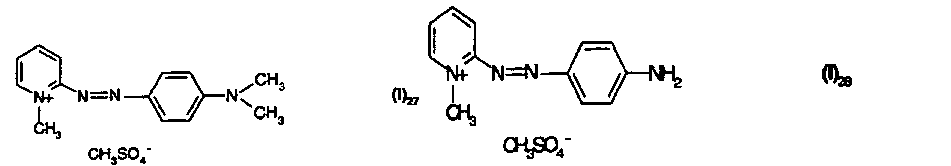

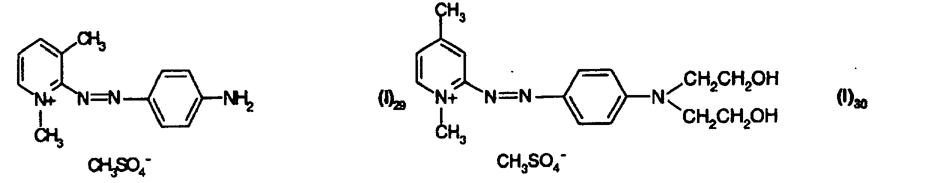

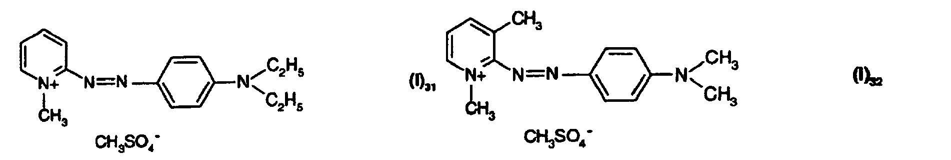

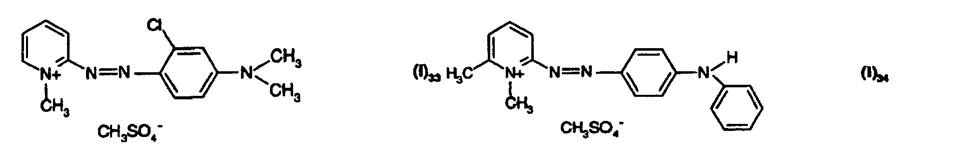

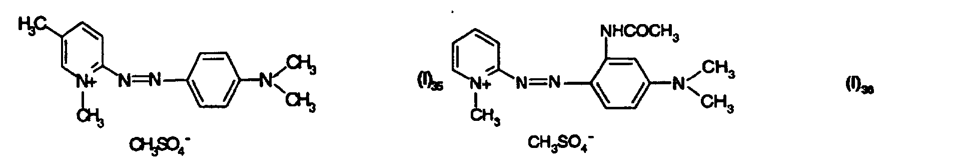

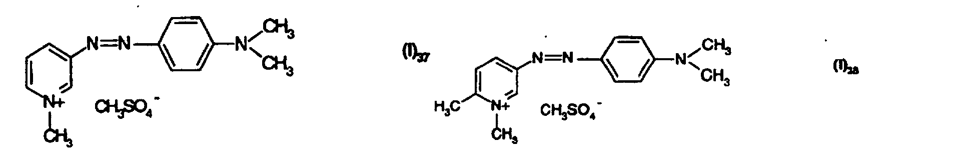

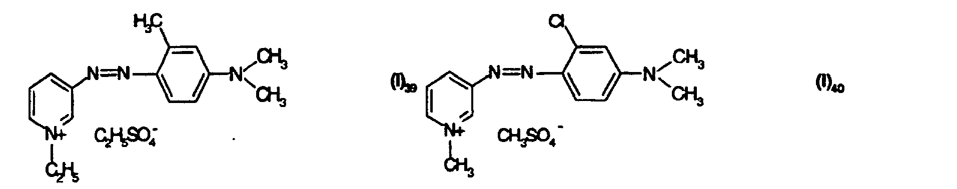

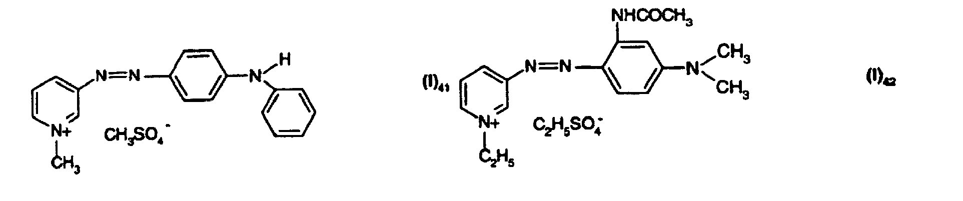

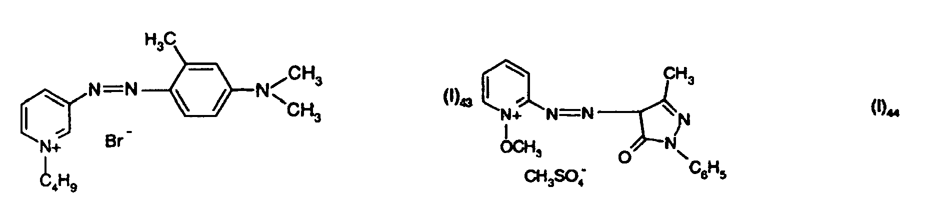

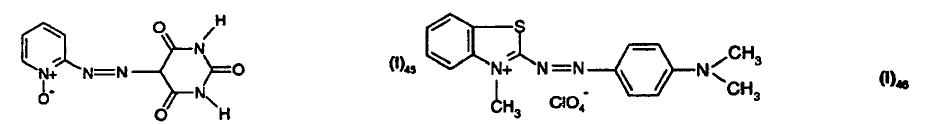

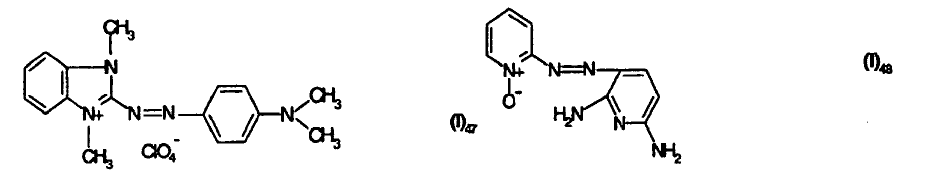

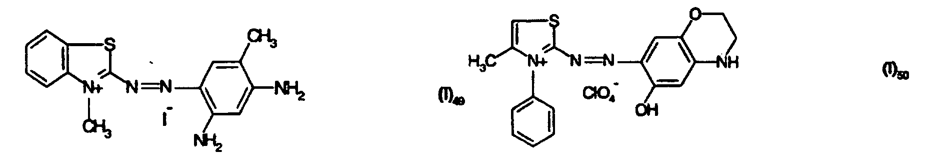

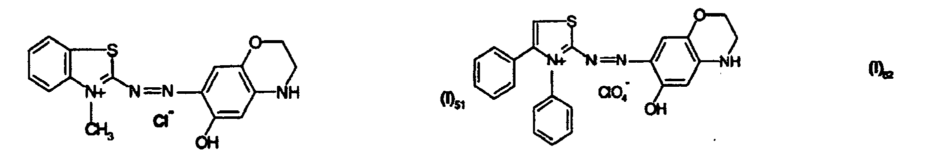

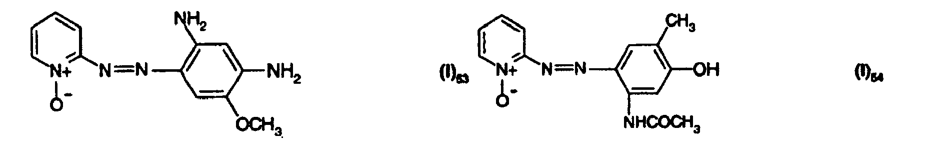

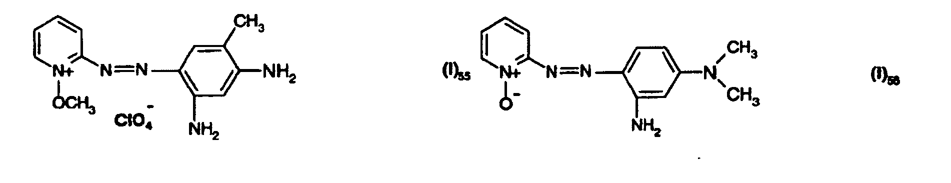

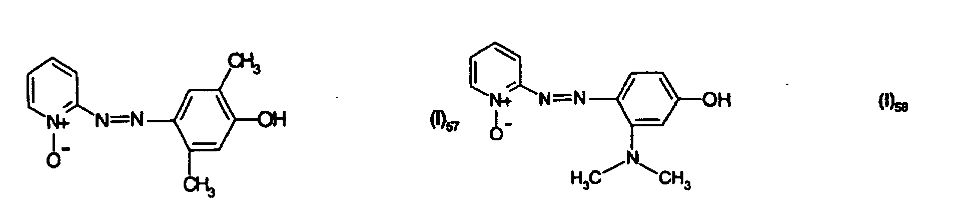

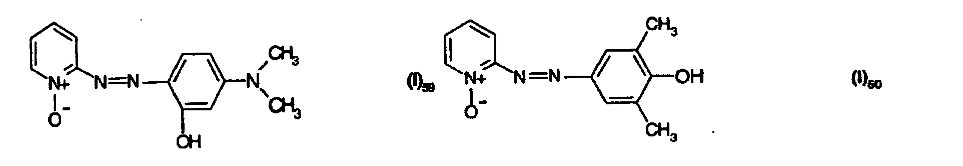

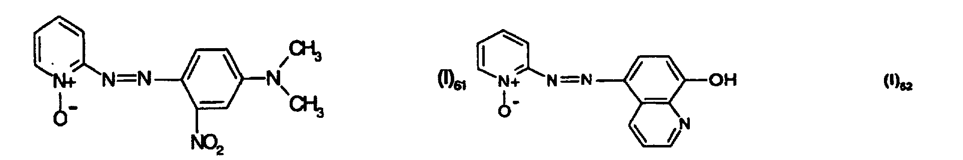

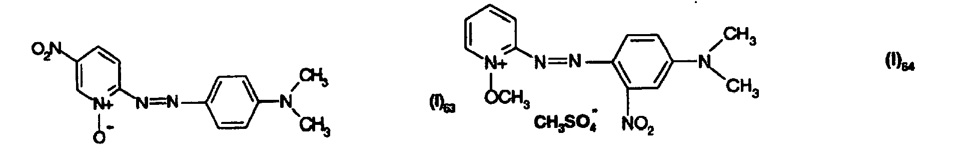

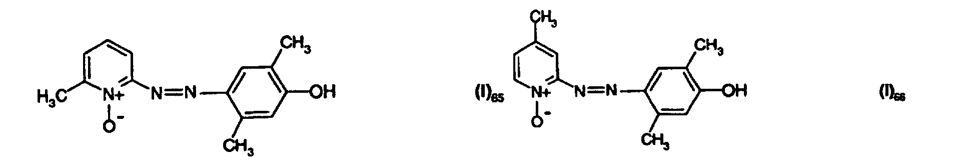

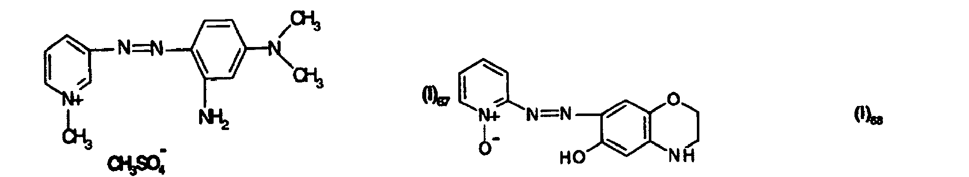

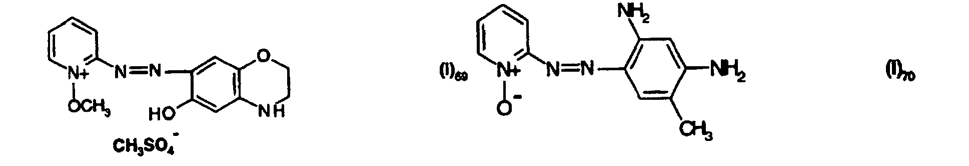

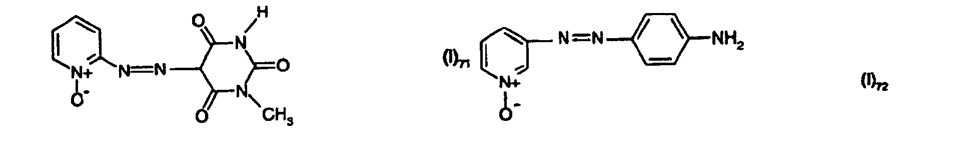

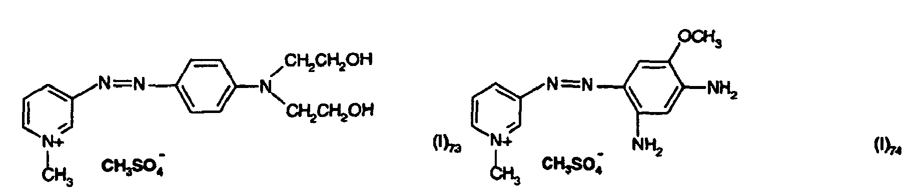

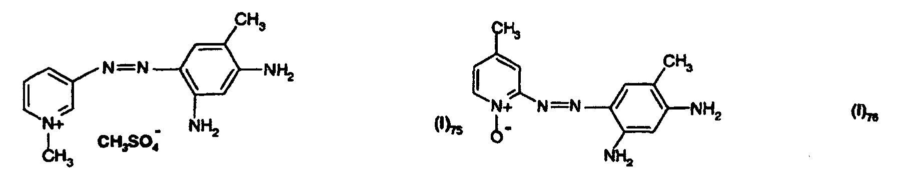

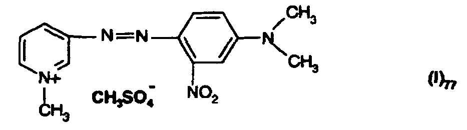

Le complexe utile dans la présente invention comprend de préférence une quantité d'acide et de colorant direct telle que rapport massique acide/colorant est supérieur à 1/100, de préférence supérieur à 1/30.

La quantité de complexe dans la composition oxydante est comprise entre 0,5 et 50 % en poids par rapport au poids de composition oxydante, de préférence entre 1 et 40 %.

Selon un mode de préparation particulier du complexe, l'acide et le colorant direct cationique sont mis en solution dans un solvant organique approprié. Dans cette étape l'acide vient complexer le colorant direct cationique formant ainsi une protection pour le colorant direct cationique. La solution ainsi obtenue est coulée dans de l'eau sous agitation afin de précipiter le complexe formé de l'acide et du colorant cationique sous forme solide. Après lavage, filtration et séchage, on obtient le complexe sous forme d'une poudre.

La composition, oxydante de l'invention est obtenue par mélange de cette poudre avec un agent oxydant en solution dans un milieu approprié à la teinture.

L'agent oxydant peut être n'importe quel agent oxydant classique dans le domaine de la coloration. A titre d'exemple, l'agent oxydant est choisi parmi le peroxyde d'hydrogène, le peroxyde d'hydrogène, le peroxyde d'urée, les bromates de métaux alcalins, les persels tels que les perborates et persulfates, les peracides.

Selon la présente invention, la composition alcalinisante contient un agent alcalin. Un tel agent peut être choisi parmi les agents alcalins classiques utilisés dans le domaine de la coloration. A titre d'exemple, l'agent alcalin est choisi parmi l'ammoniaque, les carbonates alcalins, les alcanolamines, les hydroxydes de sodium
ou de potassium et les composés de formule (III) suivante : 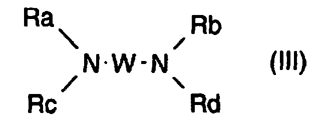
dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition alcalinisante peut de plus comprendre une ou plusieurs bases d'oxydation choisies parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques ainsi que leurs sels d'addition.

Parmi les paraphénylènediamines utilisables à titre de base d'oxydation dans les compositions tinctoriales conformes à l'invention, on peut notamment citer les composés de formule (1b) suivante et leurs sels d'addition avec un acide : 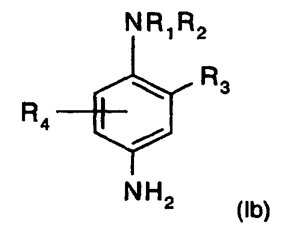
dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄)
ou alkyle en C₁-C₄ substitué par un groupement azoté ;
- R₁, R₂ et l'atome d'azote auquel ils sont rattachés peuvent former un hétérocycle,
- R₃ représente un atome d'hydrogène, un atome d'halogène tel qu'un atome de chlore, de brome, d'iode ou de fluor, un radical alkyte en C₁-C₄, monohydroxyalkyle en C₁-C₄, hydroxyaicoxy en C₁-C₄, acétylaminoalcoxy en C₁-C₄, mésytaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (1b) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β, γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, la 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bases doubles utilisables à titre de bases d'oxydation dans les compositions tinctoriales conformes à l'invention, on peut notamment citer les composés répondant à la formule (IIb) suivante, et leurs sels d'addition avec un acide : 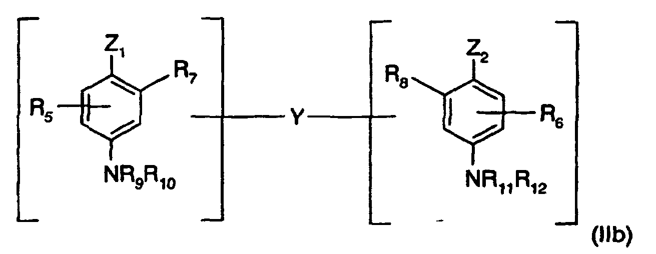
dans laquelle :
- Z₁ et Z₂, identiques ou différents, représentent un radical hydroxyle ou - NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈ R₉, R₁₀ R₁₁ et R₁₂. identiques ou différents, représentent un atome d'hydrogène, un bras de liaison Y ou un radical alkyle en C₁-C₄ ;

étant entendu que les composés de formule (IIb) ne comportent qu'un seul bras de liaison Y par molécule.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide. On peut aussi utiliser des bases doubles paraaminophénols.

Parmi les para-aminophénols utilisables à titre de bases d'oxydation dans les compositions tinctoriales conformes à l'invention, on peut notamment citer les composés répondant à la formule (IIIb) suivante, et leurs sels d'addition avec un acide : 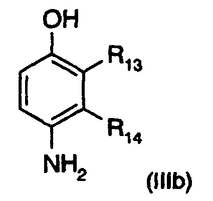
dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁₋C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁₋C₄, cyanoalkyle en C₁-C₄ ou alcoxy(C₁-C₄)alkyle(C₁-C₄). étant entendu qu'au moins un des radicaux R₁₃ ou R₁₄ représente un atome d'hydrogène.

Parmi les para-aminophénols, on peut citer à titre d'exemple, le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamin ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]éthanol, la 5,6-diméthyt pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation additionnelles sont présentes en quantité de préférence comprise entre 0,001 à 10 % en poids environ du poids total de la composition tinctoriale, et encore plus préférentiellement de 0,005 à 6 %.

La composition alcalinisante utile dans la présente invention peut de plus contenir un ou plusieurs coupleurs additionnels qui sont conventionnellement utilisés pour la teinture de fibres kératiniques: Parmi ces coupleurs, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthytène dioxybenzène, le 2,6-bis-(β-hydroxyéthylamino)toluène et leurs sels d'addition.

Dans la composition de la présente invention, le ou les coupleurs sont présents en quantité de préférence comprise entre 0,001 et 10 % en poids environ du poids total de la composition et encore plus préférentiellement de 0,005 à 6 %.

D'une manière générale, les sels d'addition utiles dans le cadre de la composition de l'invention pour les bases d'oxydation et les coupleurs présents sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les les sels d'addition avec une base telles que la soude, la potasse, l'ammoniaque, les amines ou les alcanolamines.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le milieu approprié pour la teinture peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas ou substantiellement pas, altérées par la ou les adjonctions envisagées.

L'invention a également pour objet un procédé de teinture des fibres kératiniques et en particulier des fibres kératiniques humaines telles que les cheveux mettant en oeuvre le kit de la présente invention.

Selon ce procédé, on mélange la composition oxydante et la composition alcalinisante pour former une composition prête à l'emploi. Cette composition prête à l'emploi est ensuite appliquée sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Avant application sur les fibres, il peut être nécessaire de modifier le pH de la composition prête à l'emploi au moyen d'agents acidifiants ou alcalinisants habituellement utilisés en teinture des fibres kératiniques et tels que définis précédemment. Le pH de la composition prête à l'emploi peut être ajusté à des pH variants de 3 à 12, de préférence de 5 à 11.

Un autre objet de l'invention est un dispositif à plusieurs compartiments dans lequel un premier compartiment renferme la composition alcalinisante définie ci-dessus et un deuxième compartiment renferme la composition oxydante définie ci dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

### Exemple 1 :

### Préparation du complexe

3g d'acide chenodeoxycholique et 50 mg de colorant (V14) sont introduits dans un flacon de 50ml. On ajoute ensuite 10g d'éthanol absolu, 5g d'acétone et on place le mélange sous agitation 30 min. La solution obtenue est coulée dans 500 ml d'eau sous agitation. II se forme un précipité qui est récupéré par filtration sur fritté N°3. Après lavage à l'eau du précipité (500 ml) sous agitation, filtration et séchage, on récupère 2,4g de matière sèche.

### Préparation de la composition oxydante:

0,5g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à une composition (A) comprenant

| | |
|---|---|
| paratoluènediamine | 0,2 g |
| résorcine | 0,1 g |
| paraaminophénol | 0,5 g |
| 2-méthyl 5-β-hydroxyéthylaminophénol | 0,35 g |
| 2-méthyl 5-amino phénol | 0,3 g |
| Acide oléique | 2,7 g |
| Monoéthanolamine | 0,6 g |
| Alcool cétylstéarylique | 16 g |
| Alcool oléocétylique à 30 moles d'OE | 3,5 g |
| séquestrant | 0,8 g |
| Ammoniaque à 20,5 % de NH3 | 10,3 g |
| Thiolactate d'ammonium | 4,8 g |
| Parfum | qs |
| Eau déminéralisée | qsp 100 g |

La poudre se dissout immédiatement pour donner une composition colorée dans laquelle le colorant est maintenant disponible pour la teinture des cheveux.

### Exemple 2 :

### Préparation du complexe:

3g d'acide chenodeoxycholique et 50mg de colorant (V2) sont introduits dans un flacon de 50m1. On ajoute ensuite 10g d'éthanol absolu et on place le mélange sous mettre sous agitation 30 min. La solution obtenue est coulée dans 500 ml d'eau sous agitation. II se forme un précipité qui est récupéré par filtration sur fritté N°3. Après lavage dans 500 ml d'eau sous agitation du précipité, filtration et séchage, on récupère 2,4g de matière sèche.

### Préparation de la composition oxydante:

0,5g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 3:

### Préparation du complexe

3g d'acide chenodeoxycholique et 50mg de colorant (VII4) sont introduits dans un flacon de 50ml. On ajoute ensuite 10g d'éthanol absolu et on place le mélange sous agitation 30 min. La solution obtenue est coulée dans 500 ml d'eau sous agitation. Il se forme un précipité. Il se forme un précipité qui est récupéré par filtration sur fritté N°3. Après lavage à l'eau du précipité (500 ml) sous agitation, filtration et séchage, on récupère 2,4g de matière sèche.

### Préparation de la composition oxydante:

0,5g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 4 :

### Préparation du complexe

Dans un bécher, on introduit 10mg de colorant (V14) et 1g d'acide benzoïque en fusion (125°C). On laisse refroidir jusqu'à la température ambiante (15 minutes). On répète deux fois l'addition de l'acide benzoïque en fusion (125°C). On récupère 3g de matière sèche.

### Préparation de la composition oxydante.

0,5g de la matière sèche obtenue ci dessus sont mis en suspension dans 10ml de H₂O₂ 12vol. La poudre est stable dans la solution de peroxyde. Elle est insoluble dans la solution et la couleur de la poudre reste inchangée après 2 mois de conservation à 45°C

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 5 :

### Préparation du complexe

Dans un flacon de 30 ml, on introduit 3g d'acide chenodeoxycholique. On porte l'acide chenodeoxycholique en fusion (170°C). On ajoute ensuite 50mg de colorant (V2) sous agitation. Une fois la totalité du colorant dissout, on laisse refroidir jusqu'à température ambiante (60 min). On récupère 3g de matière sèche.

### Préparation de la composition oxydante:

0.5g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 6 :

### Préparation du complexe

Dans un flacon de 50 ml, on introduit 3g d'acide chenodeoxycholique et 50 mg du colorant suivant :

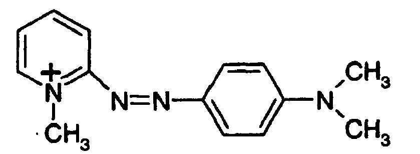

On ajoute ensuite 10 g d'éthanol absolu et on agite pendant 30 min. La solution obtenue est coulée dans 500 ml d'eau sous agitation. Il se forme un précipité qui est récupéré par filtration sur fritté N°3. Un lavage du précipité est effectué dans 500 ml d'eau sous agitation. Après filtration puis séchage, on récupère environ 2,4g de matière sèche.

### Préparation de la composition oxydante:

0,5 g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 7 :

### Préparation du complexe

Dans un flacon de 50 ml, on introduit 3g d'acide chenodeoxycholique et 50 mg du colorant suivant :

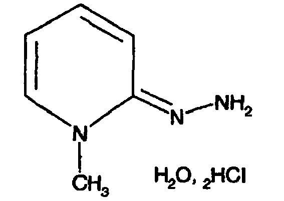

On ajoute ensuite 10 g d'éthanol absolu et on agite pendant 30 min. La solution obtenue est coulée dans 500 ml d'eau sous agitation à 50 °C. Il se forme un précipité qui est récupéré par filtration sur fritté N°3. Un lavage du précipité est effectué dans 500 ml d'eau sous agitation. Après filtration puis séchage, on récupère environ 2,4g de matière sèche.

### Préparation de la composition oxydante:

0,5 g de la matière sèche obtenue ci dessus est mis en suspension dans 10mf de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 8 :

### Préparation du complexe

Dans un flacon de 50 ml, on introduit 3g d'acide chenodeoxycholique et 50 mg du colorant suivant :

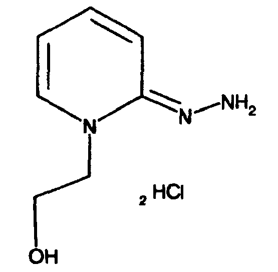

On ajoute ensuite 10 g d'éthanol absolu et on agite pendant 30 min. La solution obtenue est coulée dans 500 ml d'eau sous agitation à 50°C. Il se forme un précipité qui est récupéré par filtration sur fritté N°3. Un lavage du précipité est effectué dans 500 ml d'eau sous agitation. Après filtration puis séchage, on récupère environ 2,4g de matière sèche.

### Préparation de la composition oxydante:

0,5 g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

### Exemple 9 :

### Préparation du complexe

Dans un flacon de 50 ml, on introduit 3g d'acide chenodeoxycholique et 50 mg du colorant suivant :

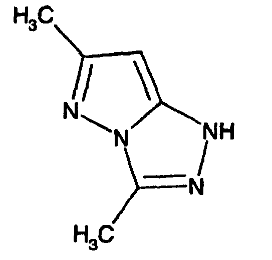

On ajoute ensuite 10 g d'éthanol absolu et on agite pendant 30 min. La solution obtenue est coulée dans 500 ml d'eau sous agitation à 50 °C. II se forme un précipité qui est récupéré par filtration sur fritté N°3. Un lavage du précipité est effectué dans 500 ml d'eau sous agitation. Après filtration puis séchage, on récupère environ 2,4g de matière sèche.

### Préparation de la composition oxydante:

0,5 g de la matière sèche obtenue ci dessus est mis en suspension dans 10ml de H₂O₂ 12vol. La poudre est stable dans la solution de peroxyde. Elle est insoluble dans la solution et la couleur de la poudre reste inchangée après 2 mois de conservation à 45°C.

### Préparation de la composition prête à l'emploi

La suspension de poudre dans le peroxyde est ajoutée à la composition (A) décrite ci dessus. La poudre se dissout immédiatement pour donner une composition colorée dans laquelle le colorant est maintenant disponible pour la teinture des cheveux.

## Revendications

1. Kit pour la teinture de fibres kératiniques comprenant
• une composition alcalinisante comprenant, dans un milieu approprié pour la teinture, un agent alcalin, et
• une composition oxydante ayant un pH inférieur ou égal à 7 comprenant, dans un milieu approprié, un agent oxydant et un complexe moléculaire en suspension dans le milieu, le complexe étant formé d'un colorant direct et d'un acide organique insoluble ou faiblement soluble dans la composition oxydante dont le pKa est supérieur au pH de la composition oxydante et inférieur au pH du mélange de la composition oxydante et de la composition alcalinisante, à la condition que l'écart entre le pKa de l'acide et le pH de la composition oxydante est d'au moins une unité et l'écart entre le pKa de l'acide et le pH du mélange est d'au moins une unité.

2. Kit selon la revendication 1 dans lequel le pH de la composition alcalinisante est compris entre 7 et 12 et le pH de la composition oxydante est inférieur ou égal à 5.

3. Kit selon la revendication 1 ou 2 dans lequel l'acide formant le complexe est choisi parmi l'acide chenodeoxycholique, l'acide benzoïque, l'acide cinnamique, l'acide cholique et l'acide desoxychotique.

4. Kit selon l'une quelconque des revendications 1 à 3 dans lequel le colorant direct est un colorant direct cationique.

5. Kit selon la revendication 4 dans lequel le colorant direct cationique est porteur d'un groupe ammonium quaternaire.

6. Kit selon la revendication 5 dans lequel le colorant direct cationique est choisi parmi les colorants les amino-anthraquinoniques cationiques, les mono- ou di-azoïques cationiques, les méthiniques cationiques, les azométhiniques cationiques ou les naphtoquinones cationiques.

7. Kit selon la revendication 6 dans lequel le colorant direct cationique est choisi parmi les colorants azoïques, azométhiniques ou méthiniques comportant un groupe ammonium quaternaire.

8. Kit selon l'une quelconque des revendications précédentes dans lequel le colorant direct cationique est un composé de formule (I) suivante : 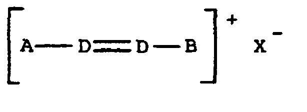 dans laquelle, D désigne un atome d'azote ou un radical CH , A et B désignent des groupements aromatiques pouvant être formés d'un cycle aromatique ayant de 4 à 6 chaînons ou de plusieurs cycles aromatiques condensés, le ou les cycles aromatiques pouvant contenir un ou plusieurs hétéroatomes et pouvant être substitués par un ou plusieurs atomes d'halogène ou par un ou plusieurs radicaux choisis parmi NR₁₁R₁₂, ou OR₁₁, dans lesquels R₁₁ et R₁₂, simultanément ou indépendamment l'un de l'autre, représentent l'hydrogène, un radical alkyle en C₁-C₈, un radical hydroxyalkyle en C₁-C₄, ou un radical phényle, et X désigne un anion, à la condition qu'au moins un des groupes A ou B comporte un groupement cationique.

9. Kit selon la revendication 8 dans lequel le groupement cationique est un groupe ammonium quaternaire.

10. Kit selon l'une quelconque des revendications précédentes 1 to 7dans lequel le colorant direct est choisi parmi les composés de formule (V) suivante : 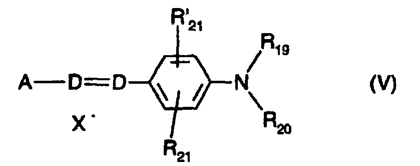
dans laquelle :
- D représente un atome d'azote ou le groupement -CH,
- R₁₉ et R₂₀, identiques ou différents, représentent un atome d'hydrogène ; un radical alkyle en C₁-C₄ pouvant être substitué par un radical -CN, -OH ou -NH₂ ; un radical 4'-aminophényle ; ou forment avec un atome de carbone du cycle benzénique un hétérocycle éventuellement oxygéné et pouvant être substitué par un ou plusieurs radicaux alkyle en C₁-C₄ ;
- R₂₁ et R'₂₁, identiques ou différents, représentent un atome d'hydrogène ou d'halogène, un radical cyano, alkyle, alcoxy en C₁-C₄ ou acétyloxy,
- X est un anion,
- A représente un groupement choisi par les structures A1 à A19 suivantes : 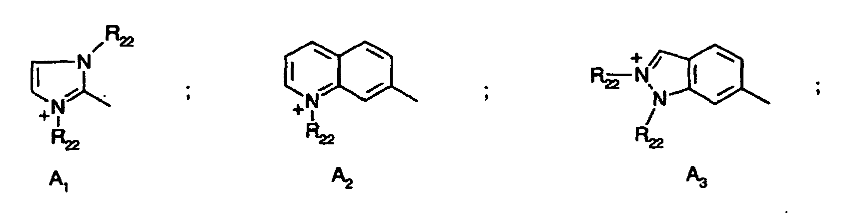 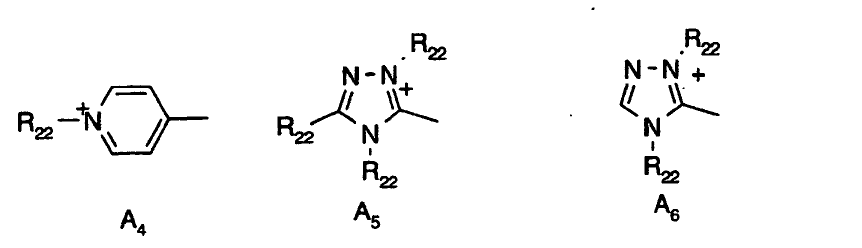 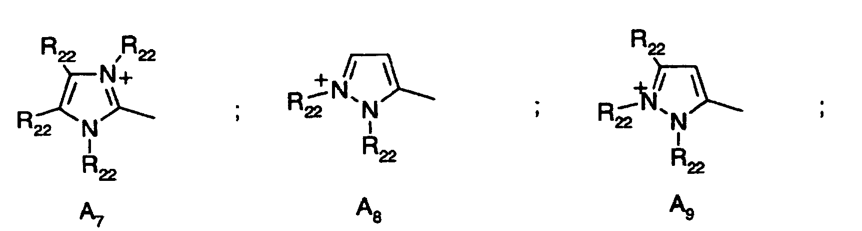 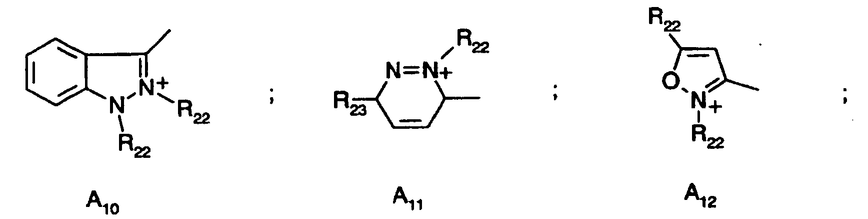 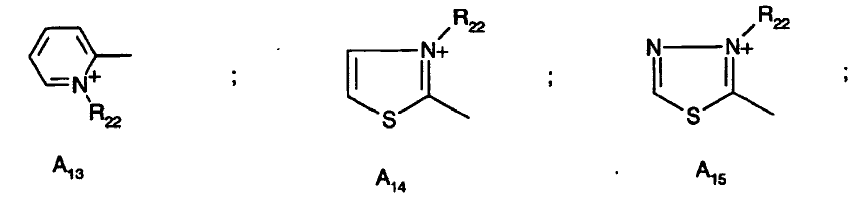 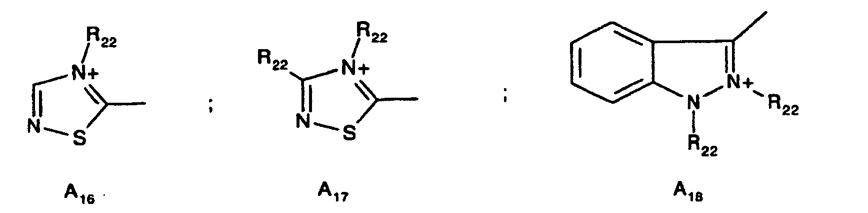 et 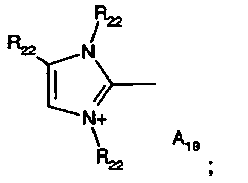
dans lesquelles
- R₂₂ représente un radical alkyle en C₁-C₄ pouvant être substitué par un radical hydroxyle et R₂₃ représente un radical alcoxy en C₁-C₄

11. Kit selon la revendication 10 dans lequel A est A1, R₁₉ et R₂₀, identiques ou différents, représentent un atome d'hydrogène, un radical alkyle en C₁-C₄ ou un radical 4'-aminophényle et R₂₂ représente un radical alkyle en C₁-C₄.

12. Kit selon l'une quelconque des revendications 1 à 9 dans lequel le colorant direct cationique est choisi parmi les composés de formule (VI) suivante : 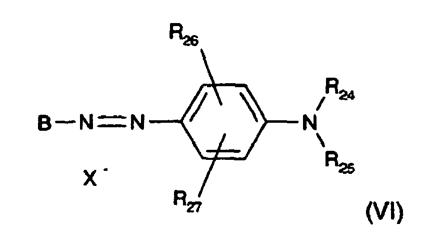
dans laquelle :
- R₂₄ représente un atome d'hydrogène ou un radical alkyle en C₁-C₄,
- R₂₅ représente un atome d'hydrogène, un radical alkyle pouvant être substitué par un radical -CN ou par un groupement amino, un radical 4'-aminophényle ou forme avec R₂₄ un hétérocycle éventuellement oxygéné pouvant être substitué par un radical alkyle en C₁-C₄,
- R₂₆ et R₂₇, identiques ou différents, représentent un atome d'hydrogène, un atome d'halogène, un radical alkyle en C₁-C₄ ou alcoxy en C₁-C₄, un radical -CN,
- X est un anion,
- B représente un groupement choisi par les structures B1 à B6 suivantes : 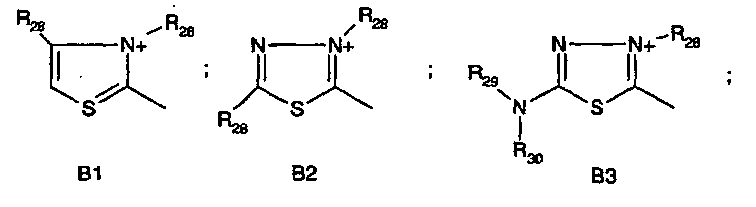 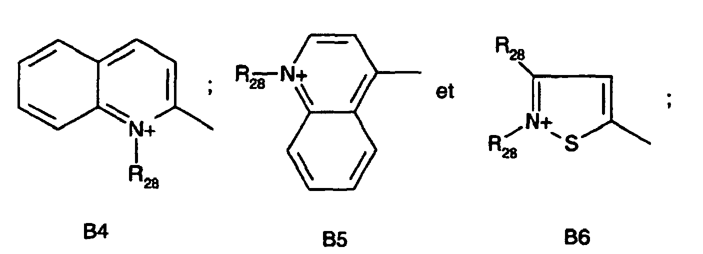
dans lesquelles R₂₈ représente un radical alkyle en C₁-C₄, R₂₉ et R₃₀, identiques ou différents, représentent un atome d'hydrogène ou un radical alkyle en C₁-C₄.

13. Kit selon l'une quelconque des revendications 1 à 7 dans lequel le colorant direct cationique est choisi parmi les composés de formules (VII) et (VII') suivantes : 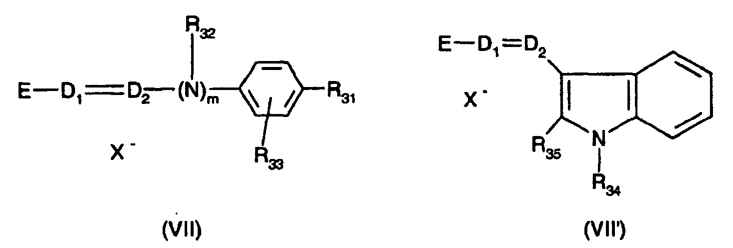
dans lesquelles :
- R₃₁ représente un atome d'hydrogène, un radical alcoxy en C₁-C₄, un atome d'halogène ou un radical amino,
- R₃₂ représente un atome d'hydrogène, un radical alkyle en C₁-C₄ ou forme avec un atome de carbone du cycle benzénique un hétérocycle éventuellement oxygéné pouvant être substitué par un ou plusieurs groupements alkyle en C₁-C₄,
- R₃₃ représente un atome d'hydrogène ou d'halogène,
- R₃₄ et R₃₅, identiques ou différents, représentent un atome d'hydrogène ou un radical alkyle en C₁-C₄,
- D₁ et D₂, identiques ou différents, représentent un atome d'azote ou le groupement - CH,
- m = 0 ou 1,
étant entendu que lorsque R₃₁, représente un groupement amino non substitué, alors D₁ et D₂ représentent simultanément un groupement -CH et m = 0,
- X est un anion,
- E représente un groupement choisi par les structures E1 à E8 suivantes : 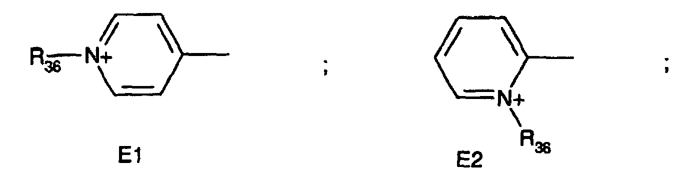 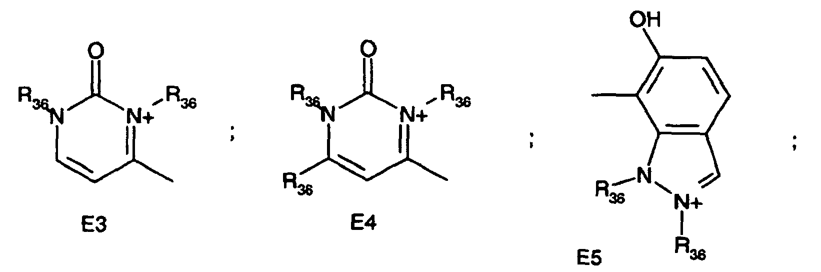 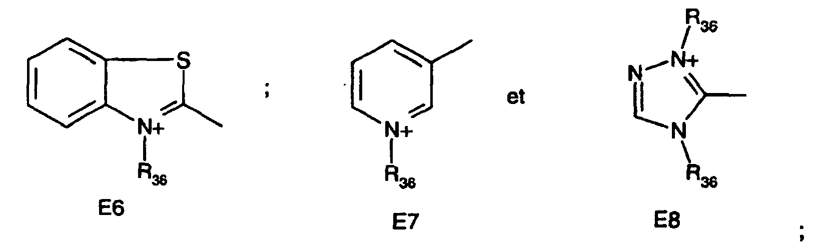
dans lesquelles R₃₆ représente un radical alkyle en C₁-C₄ ;
lorsque m = 0 et que D₁ représente un atome d'azote, alors E peut également désigner un groupement de structure E9 suivante :

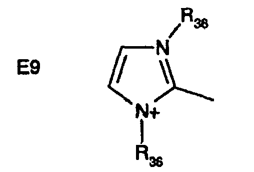

14. Kit selon la revendication 13 dans lequel le composé de formule (VII) est tel que m = 1, R₃₂ représente un atome d'hydrogène ou un radical alkyle et E représente E1.

15. Kit selon l'une quelconque des revendications précédentes dans lequel le colorant direct est choisi parmi les colorants

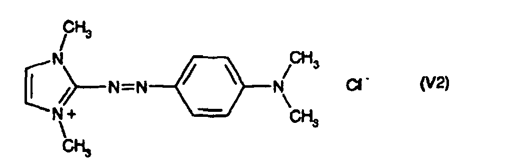

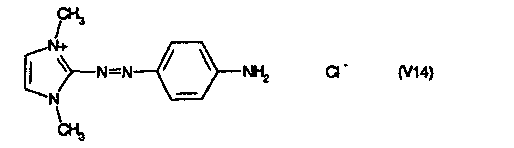

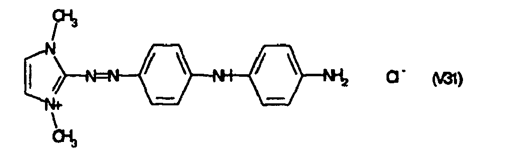

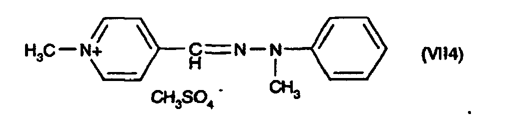

16. Kit selon l'une quelconque des revendications précédentes dans lequel l'acide et le colorant direct formant le complexe sont présent en quantité telle que le rapport massique acide/colorant est supérieur à1/100 de préférence 1/30 et la quantité de complexe dans la composition oxydante est comprise entre 0,5 et 50 % en poids par rapport au poids de composition oxydante, de préférence entre 1 et 40 %.

17. Kit selon l'une quelconque des revendications précédentes dans lequel l'agent oxydant est choisi parmi le peroxyde d'hydrogène, le peroxyde d'hydrogène, le peroxyde d'urée, les bromates de métaux alcalins, les persels et les peracides.

18. Kit selon l'une quelconque des revendications précédentes dans lequel l'agent alcalin est choisi parmi l'ammoniaque, les carbonates alcalins, les alcanolamines, les hydroxydes de sodium ou de potassium et les composés de formule (III) suivante : 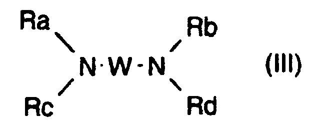
dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyatkyte en C₁-C₄.

19. Kit selon l'une quelconque des revendications précédentes dans lequel la composition alcalinisante contient de plus une ou plusieurs bases d'oxydation choisies parmi les paraphénylènediamines, les bis-phénylalkylènediamines, les para-aminophénols, les ortho-aminophénols et les bases hétérocycliques ainsi que leurs sels d'addition.

20. Kit selon la revendication 19 dans lequel la composition alcalinisante contient une quantité de base d'oxydation comprise entre 0,001 et 10 %, de préférence entre 0,005 et 6 %.

21. Kit selon la revendication 19 ou 20 dans lequel la composition alcalinisante contient de plus un ou plusieurs coupleurs choisis parmi les métaphénylènediamines, les méta-aminophénols, les métadiphénols, les coupleurs naphtaléniques et les coupleurs hétérocycliques ainsi que leurs sels d'addition.

22. Composition oxydante telle que définie dans l'une quelconque des revendications 1, 3 à 17.

23. Procédé de teinture d'oxydation des fibres kératiniques et en particulier des fibres kératiniques humaines telles que les cheveux, dans lequel on mélange la composition oxydante telle que définie dans l'une quelconque des revendications 1, 3 à 17 avec une composition alcalinisante telle que définie dans l'une quelconque des revendications 1,2 et 18 à 21, on applique ce mélange sur les fibres kératiniques et après un temps de pose, on rince les fibres.

24. Dispositif à plusieurs compartiments dans lequel un premier compartiment contient une composition alcalinisante telle que définie à l'une quelconque des revendications 1,2 et 18 à 21 et un deuxième compartiment contient une composition oxydante telle que définie dans l'une quelconque des revendications 1,3à17.

25. Composition prête à l'emploi comprenant en mélange la composition alcalinisante et la composition oxydante définies dans l'une quelconque des revendications 1 à 22.

## Claims

1. Kit for dyeing keratin fibres, comprising
• a basifying composition comprising, in a medium that is suitable for dyeing, an alkaline agent, and
• an oxidizing composition with a pH of less than or equal to 7, comprising, in a suitable medium, an oxidizing agent and a molecular complex suspended in the medium, the complex being formed from a direct dye and an organic acid that is insoluble or sparingly soluble in the oxidizing composition, the pKa of which is higher than the pH of the oxidizing composition and lower than the pH of the mixture of the oxidizing composition and of the basifying composition, with the condition that the difference between the pKa of the acid and the pH of the oxidizing composition is at least one unit and the difference between the pKa of the acid and the pH of the mixture is at least one unit.

2. Kit according to Claim 1, in which the pH of the basifying composition is between 7 and 12, and the pH of the oxidizing composition is less than or equal to 5.

3. Kit according to Claim 1 or 2, in which the acid forming the complex is chosen from chenodeoxycholic acid, benzoic acid, cinnamic acid, cholic acid and deoxycholic acid.

4. Kit according to any one of Claims 1 to 3, in which the direct dye is a cationic direct dye.

5. Kit according to Claim 4, in which the cationic direct dye bears a quaternary ammonium group.

6. Kit according to Claim 5, in which the cationic direct dye is chosen from cationic aminoanthraquinone dyes, cationic monoazo or diazo dyes, cationic methine dyes, cationic azomethine dyes and cationic naphthoquinone dyes.

7. Kit according to Claim 6, in which the cationic direct dye is chosen from azo dyes, azomethine dyes and methine dyes bearing a quaternary ammonium group .

8. Kit according to any one of the preceding claims, in which the cationic direct dye is a compound of formula (I) below: 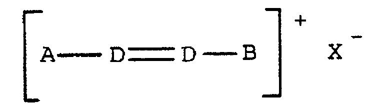
in which D denotes a nitrogen atom or a CH radical, A and B denote aromatic groups that may be formed from a 4- to 6-membered aromatic ring or from several fused aromatic rings, the aromatic ring(s) possibly containing one or more hetero atoms and possibly being substituted with one or more halogen atoms or with one or more radicals chosen from NR₁₁R₁₂ and OR₁₁, in which R₁₁ and R₁₂, simultaneously or independently of each other, represent hydrogen, a C₁-C₈ alkyl radical, a C₁-C₄ hydroxyalkyl radical or a phenyl radical, and X denotes an anion, with the condition that at least one of the groups A or B comprises a cationic group.

9. Kit according to Claim 8, in which the cationic group is a quaternary ammonium group.

10. Kit according to any one of the preceding claims 1 to 7, in which the direct dye is chosen from the compounds of formula (V) below: 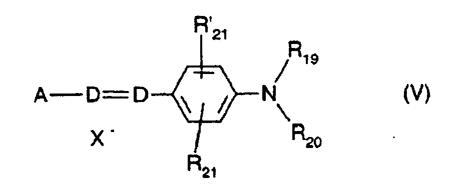
in which:
- D represents a nitrogen atom or a -CH group,
- R₁₉ and R₂₀, which may be identical or different, represent a hydrogen atom; a C₁-C₄ alkyl radical which may be substituted with a -CN, -OH or ―NH₂ radical; a 4'-aminophenyl radical; or form, with a carbon atom of the benzene ring, an optionally oxygenated heterocycle which may be substituted with one or more C₁-C₄ alkyl radicals;
- R₂₁ and R'₂₁, which may be identical or different, represent a hydrogen atom or a halogen atom or a cyano, alkyl, C₁-C₄ alkoxy, or acetyloxy radical.
- X is an anion,
- A represents a group chosen from structures A1 to A19 below: 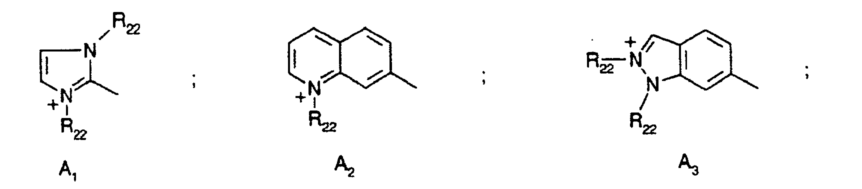 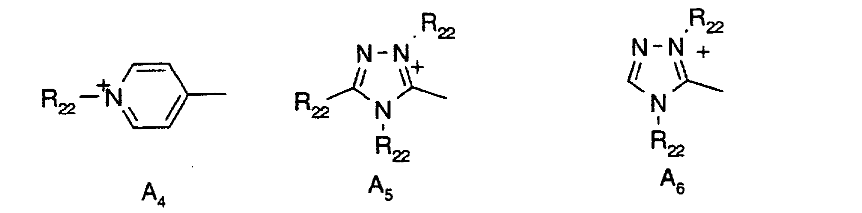 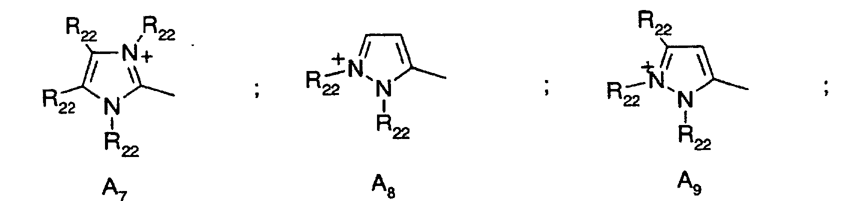 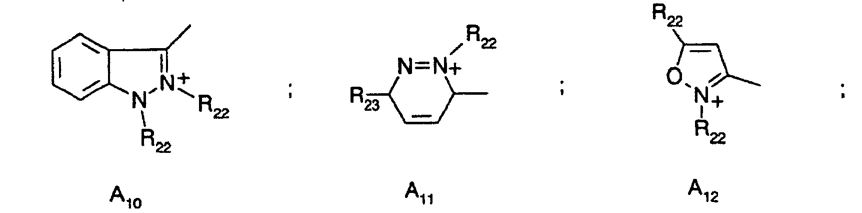 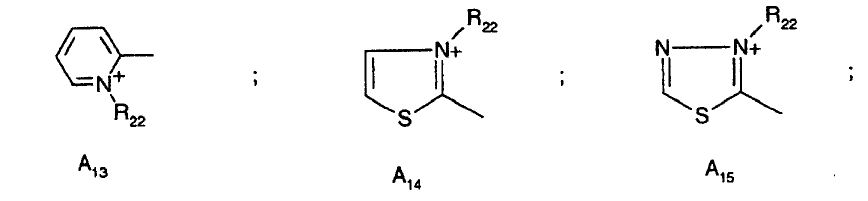 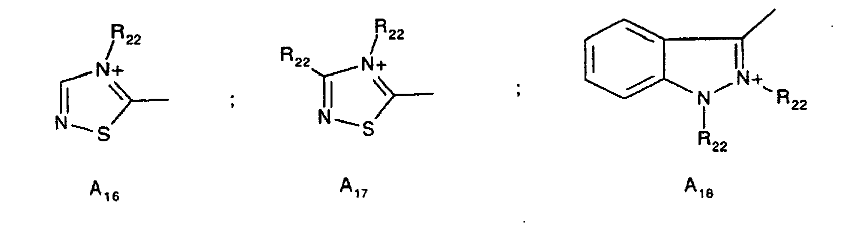
and 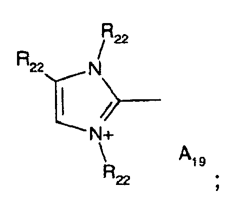
in which
- R₂₂ represents a C₁-C₄ alkyl radical which may be substituted with a hydroxyl radical, and R₂₃ represents a C₁-C₄ alkoxy radical.

11. Kit according to Claim 10, in which A is A1, R₁₉ and R₂₀, which may be identical or different, represent a hydrogen atom, a C₁-C₄ alkyl radical or a 4'-aminophenyl radical, and R₂₂ represents a C₁-C₄ alkyl radical.

12. Kit according to any one of Claims 1 to 9, in which the cationic direct dye is chosen from the compounds of formula (VI) below: 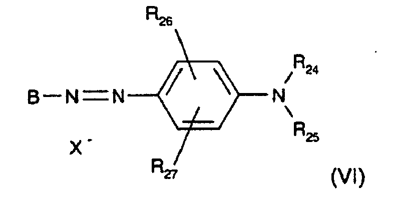
in which:
- R₂₄ represents a hydrogen atom or a C₁-C₄ alkyl radical,
- R₂₅ represents a hydrogen atom, an alkyl radical which may be substituted with a -CN radical or with an amino group, a 4'-aminophenyl radical, or forms with R₂₄ an optionally oxygenated heterocycle which may be substituted with a C₁-C₄ alkyl radical,
- R₂₆ and R₂₇, which may be identical or different, represent a hydrogen atom, a halogen atom, a C₁-C₄ alkyl or C₁-C₄ alkoxy radical, or a -CN radical,
- X is an anion,
- B represents a group chosen from the structures B1 to B6 below: 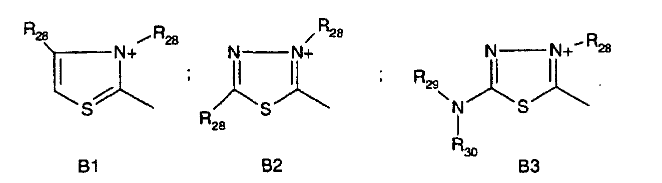 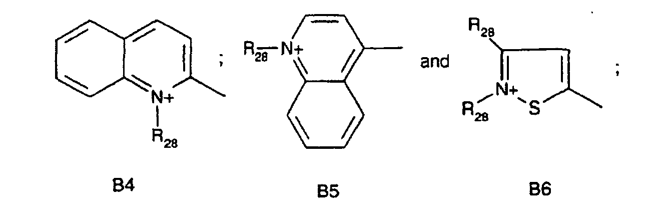
in which R₂₈ represents a C₁-C₄ alkyl radical, and R₂₉ and R₃₀, which may be identical or different, represent a hydrogen atom or a C₁-C₄ alkyl radical.

13. Kit according to any one of Claims 1 to 7, in which the cationic direct dye is chosen from the compounds of formulae (VII) and (VII') below: 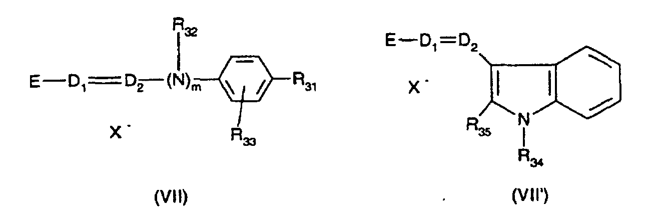
in which:
- R₃₁ represents a hydrogen atom, a C₁-C₄ alkoxy radical, a halogen atom, or an amino radical,
- R₃₂ represents a hydrogen atom, a C₁-C₄ alkyl radical or forms, with a carbon atom of the benzene ring, an optionally oxygenated heterocycle which may be substituted with one or more C₁-C₄ alkyl groups,
- R₃₃ represents a hydrogen atom or a halogen atom,
- R₃₄ and R₃₅, which may be identical or different, represent a hydrogen atom or a C₁-C₄ alkyl radical,
- D₁ and D₂, which may be identical or different, represent a nitrogen atom or a -CH group,
- m = 0 or 1,
it being understood that when R₃₁ represents an unsubstituted amino group, then D₁ and D₂ simultaneously represent a -CH group and m = 0,
- X is an anion,
- E represents a group chosen from the structures E1 to E8 below: 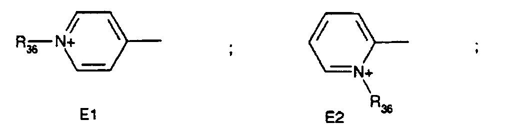 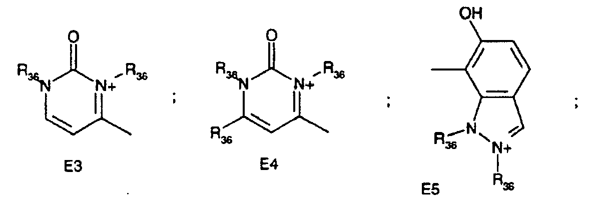 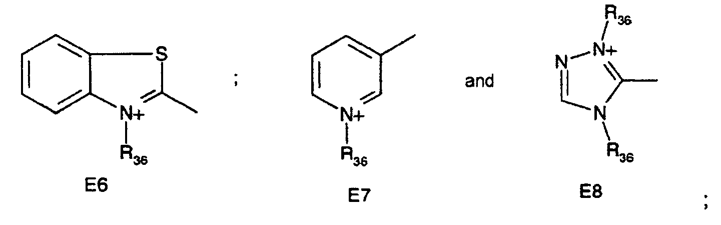
in which R₃₆ represents a C₁-C₄ alkyl radical; when m = 0 and D₁ represents a nitrogen atom, then E may also denote a group of structure E9 below:

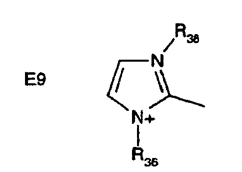

14. Kit according to Claim 13, in which the compound of formula (VII) is such that m = 1, R₃₂ represents a hydrogen atom or an alkyl radical and E represents E1.

15. Kit according to any one of the preceding claims, in which the direct dye is chosen from the dyes.

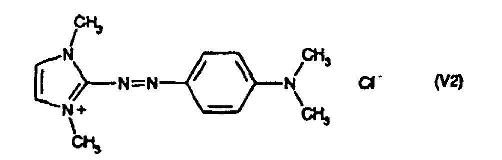

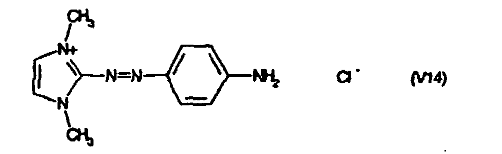

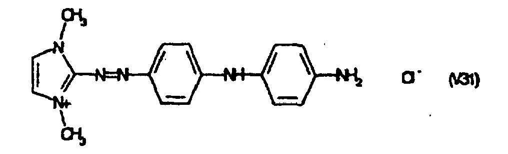

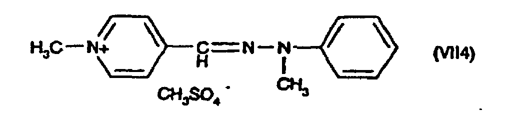

16. Kit according to any one of the preceding claims, in which the acid and the direct dye forming the complex are present in an amount such that the acid/dye mass ratio is greater than 1/100 and preferably 1/30, and the amount of complex in the oxidizing composition is between 0.5% and 50% by weight, and preferably between 1% and 40%, relative to the weight of oxidizing composition.

17. Kit according to any one of the preceding claims, in which the oxidizing agent is chosen from hydrogen peroxide, urea peroxide, alkali metal bromates, persalts and peracids.

18. Kit according to any one of the preceding claims, in which the alkaline agent is chosen from aqueous ammonia, alkali metal carbonates, alkanolamines, sodium hydroxide, potassium hydroxide and the compounds of formula (III) below: 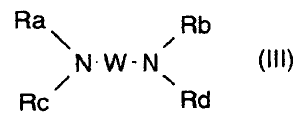
in which W is a propylene residue optionally substituted with a hydroxyl group or a C₁-C₄ alkyl radical; Rₐ, R_{b}, R_{c} and R_{d}, which may be identical or different, represent a hydrogen atom or a C₁-C₄ alkyl or C₁-C₄ hydroxyalkyl radical.

19. Kit according to any one of the preceding claims, in which the basifying composition also contains one or more oxidation bases chosen from para-phenylenediamines, bis(phenyl)alkylenediamines, para-aminophenols, ortho-aminophenols and heterocyclic bases, and also the addition salts thereof.

20. Kit according to Claim 19, in which the basifying composition contains an amount of oxidation base of between 0.001% and 10% and preferably between 0.005% and 6%.

21. Kit according to Claim 19 or 20, in which the basifying composition also contains one or more couplers chosen from meta-phenylenediamines, meta-aminophenols, meta-diphenols, naphthalene-based couplers and heterocyclic couplers, and also the addition salts thereof.

22. Oxidizing composition as defined in any one of Claims 1 and 3 to 17.

23. Process for the oxidation dyeing of keratin fibres, and in particular of human keratin fibres such as the hair, in which the oxidizing composition as defined in any one of Claims 1 and 3 to 17 is mixed with a basifying composition as defined in any one of Claims 1, 2 and 18 to 21, this mixture is applied to the keratin fibres and, after an action time, the fibres are rinsed.

24. Multi-compartment device in which a first compartment contains a basifying composition as defined in any one of Claims 1, 2 and 18 to 21, and a second compartment contains an oxidizing composition as defined in any one of Claims 1 and 3 to 17.

25. Ready-to-use composition comprising, as a mixture, the basifying composition and the oxidizing composition defined in any one of Claims 1 to 22.

## Patentansprüche

1. Kit zum Färben von Keratinfasern, das umfasst
- eine alkalisierende Zusammensetzung, die in einem zum Färben geeigneten Medium ein Alkalisierungsmittel enthält und
- durch eine oxidierende Zusammensetzung mit einem pH-Wert von 7 oder darunter, die in einem geeigneten Medium ein Oxidationsmittel und einen in dem Medium suspendierten Mol.ekülkomplex enthält, wobei der Komplex aus einem Direktfarbstoff und einer in der oxidierenden Zusammensetzung unlöslichen oder schwach löslichen Säure gebildet wird, deren pKa-Wert über dem pH-Wert der oxidierenden Zusammensetzung und unter dem pH-Wert des Gemisches der oxidierenden Zusammensetzung und der alkalisierenden Zusammensetzung liegt, mit der Maßgabe, dass der Unterschied zwischen dem pKa-Wert der Säure und dem pH-Wert der oxidierenden Zusammensetzung mindestens eine Einheit und der Unterschied zwischen dem pKa-Wert der Säure, und dem pH-Wert des Gemisches mindestens eine Einheit ist.

2. Kit nach Anspruch 1, wobei der pH-Wert der alkalisierenden Zusammensetzung im Bereich von 7 bis 12 und der pH-Wert der oxidierenden Zusammensetzung bei 5 oder darunter liegt.

3. Kit nach Anspruch 1 oder 2, wobei die Säure, die den Komplex bildet, unter Chenodesoxycholsäure, Benzoesäure, Zimtsäure, Cholsäure und Desoxycholsäure ausgewählt ist.

4. Kit nach einem der Ansprüche 1 bis 3, wobei der Direktfarbstoff ein kationischer Direktfarbstoff ist.

5. Kit nach Anspruch 4, wobei der kationische Direktfarbstoff eine quartäre Ammoniumgruppe trägt.

6. Kit nach Anspruch 5, wobei der kationische Direktfarbstoff unter den kationischen Aminoanthrachinonfarbstoffen, kationischen Mono- oder Di-Azofarbstoffen, kationischen Methinfarbstoffen, kationischen Azomethinfarbstoffen oder kationischen Naphthochinonfarbstoffen ausgewählt ist.

7. Kit nach Anspruch 6, wobei der kationische Direktfarbstoff unter den Azofarbstoffen, Azomethinfarbstoffen oder Methinfarbstof fen, die eine quartäre Ammoniumgruppe tragen, ausgewählt ist.

8. Kit nach einem der vorhergehenden Ansprüche, wobei der kationische Direktfarbstoff eine Verbindung der folgenden Formel (I) ist: 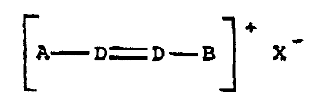
worin D ein Stickstoffatom oder CH bedeutet, A und B aromatische Gruppen sind, die aus einem 4- bis 6-gliedrigen aromatischen Ring oder mehreren kondensierten aromatischen Ringen gebildet sein können, wobei der oder die aromatischen Ringe ein oder mehrere Heteroatome enthalten können und mit einem oder mehreren Halogenatomen oder mit einer oder mehreren Gruppen substituiert sein können, die unter NR₁₁R₁₂ oder OR₁₁ ausgewählt sind, wobei R₁₁ und R₁₂ gleichzeitig oder unabhängig voneinander Wasserstoff, C₁₋₈-Alkyl, C₁₋₄-Hydroxyalkyl oder Phenyl bedeuten, und X ein Anion ist, mit der Maßgabe, dass mindestens eine der Gruppen A oder B eine kationische Gruppe enthält.

9. Kit nach Anspruch 8, wobei die kationische Gruppe eine quartäre Ammoniumgruppe ist.

10. Kit nach einem der vorhergehenden Ansprüche 1 bis 7, wobei der Direktfarbstoff unter den Verbindungen der folgenden Formel (V) ausgewählt ist: 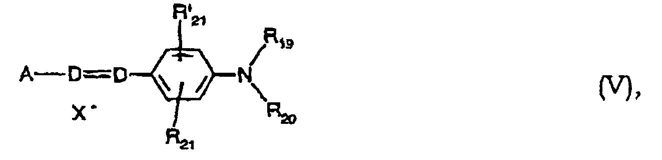
worin bedeuten:
- D ein Stickstoffatom oder die Gruppe -CH,
- die Gruppen R₁₉ und R₂₀, die gleich oder verschieden sind, ein Wasserstoffatom; eine C₁₋₄-Alkylgruppe, die mit einer Gruppe
- CN, -OH oder -NH₂ substituiert sein kann; 4'-Aminophenyl; oder sie bilden mit einem Kohlenstoffatom des Benzolrings einen gegebenenfalls sauerstoffhaltigen Heterocyclus, der mit einer oder mehreren C₁₋₄-Alkylgruppen substituiert sein kann;
- R₂₁ und R'₂₁, die gleich oder verschieden sind, ein Wasserstoffatom oder Halogenatom, Cyano, Alkyl, C₁₋₄-Alkoxy oder Acetyloxy.
- X ein Anion,
- A eine Gruppe, die unter den folgenden Strukturen A1 bis A19 ausgewählt ist: 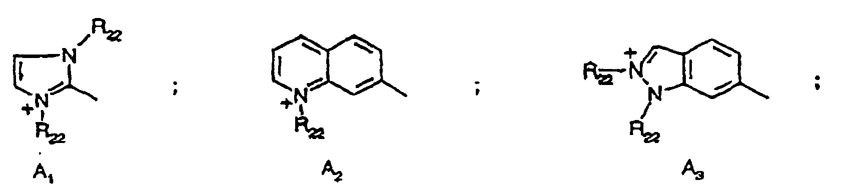 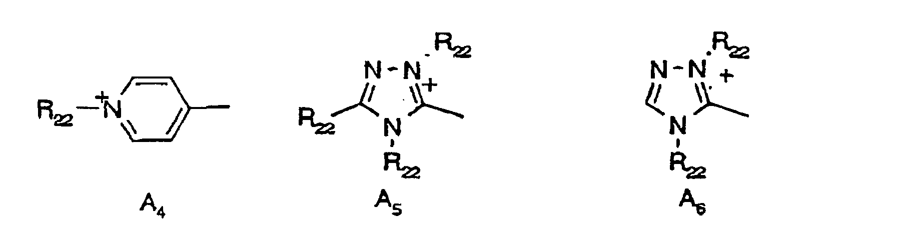 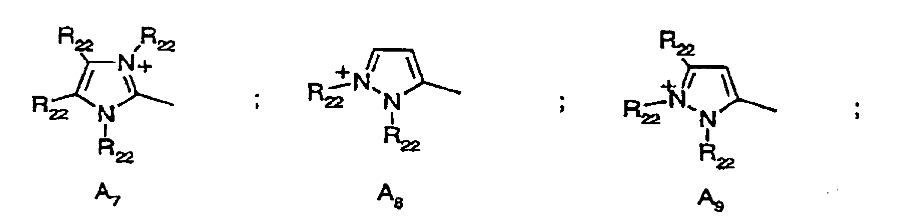 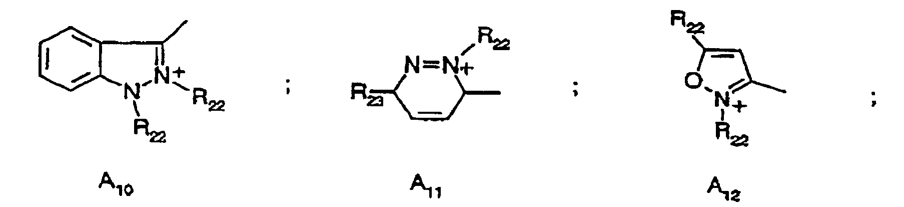 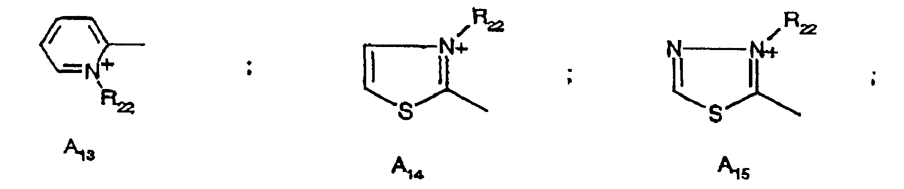 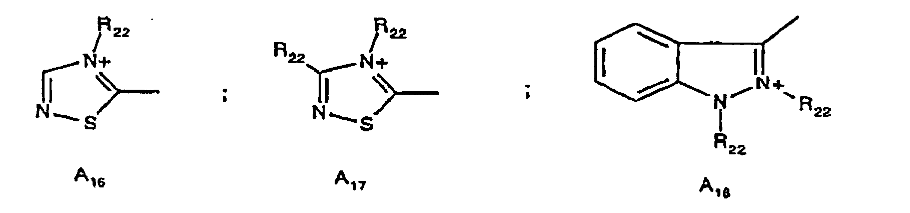
und 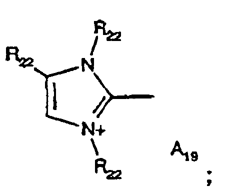
worin bedeuten
- R₂₂ eine C₁₋₄-Alkylgruppe, die mit einer Hydroxygruppe substituiert sein kann, und R₂₃ eine C₁₋₄-Alkoxygruppe.

11. Kit nach Anspruch 10, wobei A A1 bedeutet, die Gruppen R₁₉ und R₂₀, die gleich oder verschieden sind, ein Wasserstoffatom, eine C₁₋₄-Alkylgruppe oder eine 4'-Aminophenylgruppe bedeuten und R₂₂ eine C₁₋₄-Alkylgruppe ist.

12. Kit nach einem der Ansprüche 1 bis 9, wobei der kationische Direktfarbstoff unter den Verbindungen der folgenden Formel (VI) ausgewählt ist: 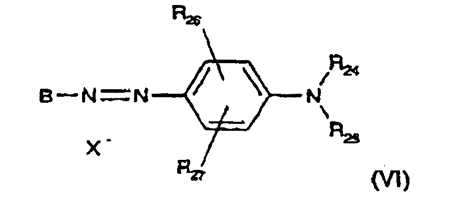
worin bedeuten:
- R₂₄ ein Wasserstoffatom oder eine C₁₋₄-Alkylgruppe,
- R₂₅ ein Wasserstoffatom, eine Alkylgruppe, die mit einer Gruppe -CN oder einer Aminogruppe substituiert sein kann,
- 4'-Aminophenyl, oder R₂₅ bildet mit R₂₄ einen gegebenenfalls sauerstoffhaltigen Heterocyclus, der mit einer C₁₋₄-Alkylgruppe substituiert sein kann,
- R₂₆ und R₂₇, die gleich oder verschieden sind, ein Wasserstoffatom, ein Halogenatom, C₁₋₄-Alkyl oder C₁₋₄-Alkoxy, oder die Gruppe -CN
- X ein Anion,
- B eine Gruppe, die unter den folgenden Strukturen B 1 bis B6 ausgewählt ist: 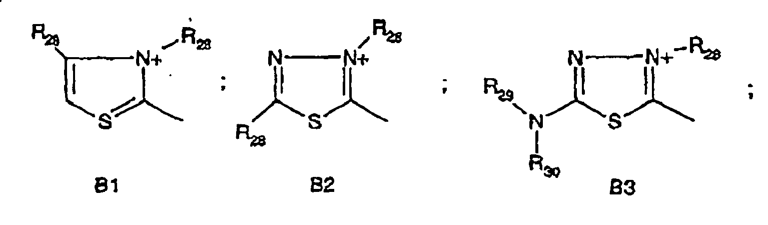 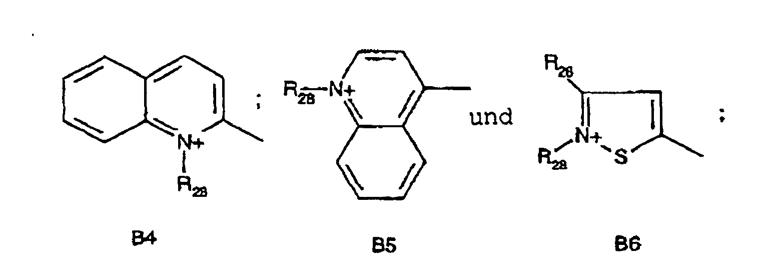
worin die Gruppen R₂₈ C₁₋₄-Alkyl bedeuten und die Gruppen R₂₉ und R₃₀, die gleich oder verschieden sind, ein Wasserstoffatom oder eine C₁₋₄-Alkylgruppe bedeuten.

13. Kit nach einem der Ansprüche 1 bis 7, wobei der kationische Direktfarbstoff unter den Verbindungen der folgenden Formeln (VII) und (VII') ausgewählt ist: 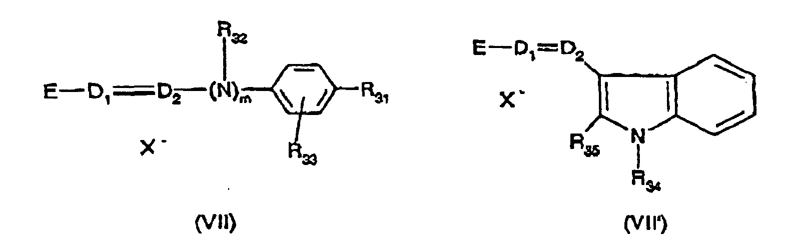
in denen bedeuten:
- R₃₁ ein Wasserstoffatom, C₁₋₄-Alkoxy, ein Halogenatom oder eine Aminogruppe,
- R₃₂ ein Wasserstoffatom, C₁₋₄-Alkyl oder R₃₂ bildet mit einem Kohlenstoffatom des Benzolrings einen gegebenenfalls sauerstoffhaltigen Heterocyclus, der mit einer oder mehreren C₁₋₄₋Alkylgruppen substituiert sein kann,
- R₃₃ ein Wasserstoffatom oder ein Halogenatom,
- R₃₄ und R_{35,} die gleich oder verschieden sind, ein Wasserstoffatom oder eine C₁-₄-Alkylgruppe,
- die D₁ und D₂, die gleich oder verschieden sind, ein Stickstoffatom oder die Gruppe -CH,
- m 0 oder 1,
mit der Maßgabe, dass D₁ und D₂ gleichzeitig eine Gruppe -CH bedeuten und m = 0, wenn R₃₁ eine unsubstituierte Aminogruppe ist,
- X ein Anion,
- E eine Gruppe, die unter den folgenden Strukturen E1 bis E8 ausgewählt ist: 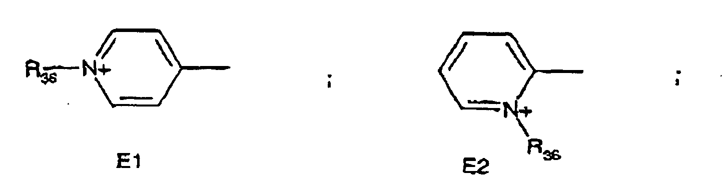 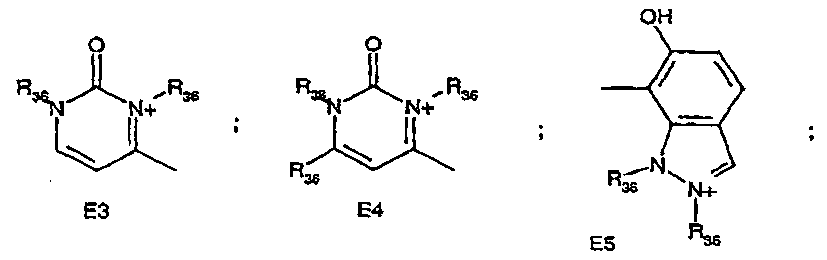 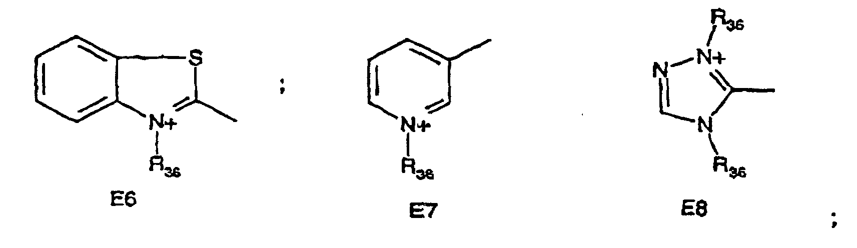
worin R₃₆ eine C₁₋₄-Alkylgruppe bedeutet;
wenn m = 0 und D₁ ein Stickstoffatom bedeutet, kann E auch eine Gruppe der folgenden Struktur E9 sein:

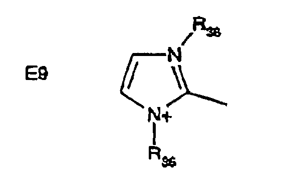

14. Kit nach Anspruch 13, wobei die Verbindung der Formel (VII) so ist, dass m = 1, R₃₂ ein Wasserstoffatom oder Alkyl bedeutet und E E1 ist.

15. Kit nach einem der vorhergehenden Ansprüche, wobei der Direktfarbstoff unter den folgenden Farbstoffen ausgewählt ist;

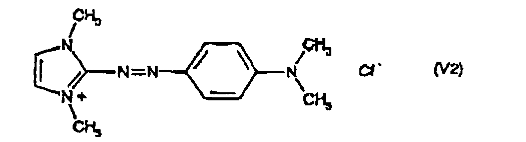

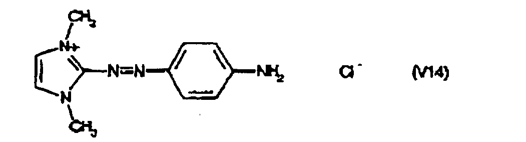

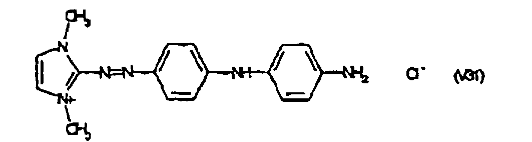

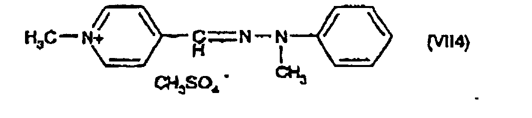

16. Kit nach einem der vorhergehenden Ansprüche, wobei die Säure und der Direktfarbstoff, die den Komplex bilden, in einer solchen Menge enthalten sind, dass das Masseverhältnis Säure/Farbstoff über 1/100 und vorzugsweise 1/30 liegt und der Mengenanteil des Komplexes in der oxidierenden Zusammensetzung im Bereich von 0,5 bis 50 Gew.-%, bezogen auf das Gesamtgewicht der oxidierenden Zusammensetzung, und vorzugsweise 1 bis 40 % liegt.

17. Kit nach einem der vorhergehenden Ansprüche, wobei das Oxidationsmittel unter Wasserstoffperoxid, Harnstoffperoxid, Alkalimetallbromaten, Salzen von Persäuren und Persäuren ausgewählt ist.

18. Kit nach einem der vorhergehenden Ansprüche, wobei das Alkalisierungsmittel unter Ammoniak, Alkalicarbonaten, Alkanolaminen, Natriumhydroxid, Kaliumhydroxid und den Verbindungen der folgenden Formel (III) ausgewählt ist: 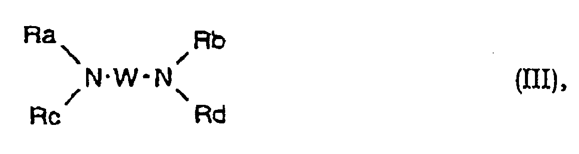
wobei W eine gegebenenfalls mit einer Hydroxygruppe oder einer C₁₋₄-Alkylgruppe substituierte Propylengruppe ist; und Rₐ, R_{b}, R_{c} und R_{d}, die gleich oder verschieden sind, ein Wasserstoffatom, C₁₋₄-Alkyl oder C₁₋₄-Hydroxyalkyl bedeuten.

19. Kit nach einem der vorhergehenden Ansprüche, wobei die alkalisierende Zusammensetzung ferner mindestens eine oder mehrere Oxidationsbasen enthält, die unter den p-Phenylendiaminen, Bis-phenylalkylendiaminen, p-Aminophenolen, o-Aminophenolen und heterocyclischen Basen sowie deren Additionssalzen ausgewählt sind.

20. Kit nach Anspruch 19, wobei die alkalisierende Zusammensetzung die Oxidationsbase in einer Menge von 0,001 bis 10 % und vorzugsweise 0,005 bis 6 % enthält.

21. Kit nach Anspruch 19 oder 20, wobei die alkalisierende Zusammensetzung ferner einen oder mehrere Kuppler enthält, die unter den m-Phenylendiaminen, m-Aminophenolen, m-Dihydroxybenzolen, Naphthalinkupplern und heterocyclischen Kupplern sowie deren Additionssalzen ausgewählt sind.

22. Oxidierende Zusammensetzung nach einem der Ansprüche 1, 3 bis 17.

23. Verfahren zum oxidativen Färben von Keratinfasern und insbesondere menschlichen Keratinfasern, wie Haaren, wobei die oxidierende Zusammensetzung nach einem der Ansprüche 1, 3 bis 17 mit einer alkalisierenden Zusammensetzung nach einem der Ansprüche 1, 2 und 18 bis 21 vermischt wird, das Gemisch auf die Keratinfasern aufgetragen wird und nach einer Einwirkzeit die Fasern gespült werden.

24. Vorrichtung mit mehreren Abteilungen, wobei eine erste Abteilung eine alkalisierende Zusammensetzung nach einem der Ansprüche 1, 2 und 18 bis 21 enthält und eine zweite Abteilung eine oxidierende Zusammensetzung nach einem der Ansprüche 1, 3 bis 17 enthält.

25. Gebrauchsfertige Zusammensetzung, die ein Gemisch der alkalisierenden Zusammensetzung und der oxidierenden Zusammensetzung enthält, die in einem der Ansprüche 1 bis 22 definiert wurden.
